# EUROPÄISCHE PATENTSCHRIFT

(11) **EP 0 781 428 B1**
(45) Veröffentlichungstag und Bekanntmachung des Hinweises auf die Patenterteilung: **11.02.1998**
(21) Anmeldenummer: 95933365.9
(22) Anmeldetag: 13.09.1995
(51) Int. Cl.: G06F 1/00, G06F 19/00

(54) **DATEN-ARCHIVIERUNGSSYSTEM**
DATA ARCHIVE SYSTEM
SYSTEME D'ARCHIVAGE DE DONNEES

(30) Priorität: 13.09.1994 DE 4432533; 15.11.1994 EP 94118018
(43) Veröffentlichungstag der Anmeldung: 02.07.1997
(73) Patentinhaber: Rost, Irmgard, D-90425 Nürnberg (DE)
(72) Erfinder: Rost, Irmgard, D-90425 Nürnberg (DE)
(74) Vertreter: Tergau, Enno, Dipl.-Ing.
(86) Internationale Anmeldenummer: EP9503597
(87) Internationale Veröffentlichungsnummer: WO9608755

(56) Entgegenhaltungen:
- WO-A-90/12464
- DE-A- 4 213 797
- FR-A- 2 680 258
- COMPUTERS & SECURITY, JUNE 1985, NETHERLANDS, Bd. 4, Nr. 2, ISSN 0167-4048, Seiten 123-134, HIGHLAND H J 'Microcomputer security: data protection techniques'

## Beschreibung

Die Erfindung betrifft ein Daten-Archivierungssystem nach dem Oberbegriff des Anspruchs 1.

In vielen Bereichen des täglichen Lebens werden persönliche Daten benötigt, die häufig jedoch nicht in gesammelter Form zur Verfügung stehen, so daß sie für jeden Bedarfsfall aufwendig zusammengesucht und -gestellt werden müssen. Oft gehen dabei Informationen verloren oder bleiben unberücksichtigt.

Lediglich exemplarisch dafür sind Patientendaten zu nennen, anhand derer nachfolgend einige Probleme bestehender Archivierungssysteme beispielhaft dargestellt werden.

Die medizinischen Daten eines Patienten werden von vielen Einrichtungen des Gesundheitssystems erhoben und dokumentiert. Bei der bislang praktizierten Dokumentation mittels schriftlicher Eintragungen in Krankenblätter, Karteikarten, Ausweise und Pässe (z.B. Diabetiker-Paß) und dem bislang praktizierten Informationsaustausch über die Versendung von Arzt- und Krankenhausentlassungsberichten gehen häufig wichtige medizinische Daten verloren.

Wechselt beispielsweise ein Patient seinen Arzt, so verbleiben die vorher gewonnenen Daten meist in der Kartei des zuletzt behandelnden Arztes und der nächste Arzt beginnt von neuem mit seiner Datensammlung. Hinzu kommt in der modernen Medizin eine zunehmende Aufsplittung der ärztlichen Versorgung in die verschiedenen Fachdisziplinen, wobei oft unter den im Einzelfall beteiligten Kollegen eine ungenügende oder sogar keinerlei Kommunikation erfolgt.

Im derzeit praktizierten Informationssystem werden daher oftmals diagnostische Maßnahmen deswegen wiederholt, weil dem nachbehandelndem Arzt über vorausgegangene Untersuchungen nur schriftlich aufgezeichnete Beurteilungen des Vorgängers vorliegen, nicht aber beispielsweise die ursprünglichen Röntgen- oder Ultraschallbilder oder Labor- und sonstigen Meßprotokolle. Auf Grund im Einzelfall unterschiedlicher Interpretationsmöglichkeiten möchte ein Arzt für seine weiteren Maßnahmen aber verständlicherweise nicht auf eine eigene Beurteilung von Originalunterlagen verzichten.

Ein weiterer gravierender Nachteil des bestehenden Informationsaustausches bei Patientendaten liegt in der Tatsache, daß wichtige medizinische Daten im Noffall oft nicht verfügbar sind. Zwar sind in der Vergangenheit eine Reihe von Pässen und Ausweisen entwickelt worden, wie z.B. der Europäische Noffall-Ausweis, der Impfausweis, der Marcumar-Ausweis, der Allergiepaß u.a., jedoch sind in nachteiliger Weise, sofern den Patienten solche Dokumente überhaupt ausgehändigt wurden, wichtige Daten auf verschiedene Papiere verteilt und Ihr Inhalt ist bei einem Verlust meist nicht rekonstruierbar.

Einen Fortschritt in der Informationstechnologie im Bereich des Gesundheitswesens könnten Patientenkarten in Form von Magnetstreifen- oder Prozessor-Chipkarten darstellen, die in der Lage sind, medizinische Informationen auf einem mobilen elektronischen Datenträger zu speichern. So sind auch in letzter Zeit einige Kartenprojekte entstanden, welche die Nutzung von Prozessor-Chipkarten zur Datenspeicherung im medizinischen Bereich zum Ziel haben.

Die bisher bekannten Entwicklungen von Patientenkarten führen aber nicht zu einer wesentlichen Verbesserung der Datenbasis für die unmittelbare Patientenbetreuung, da auf Grund der begrenzten Speicherkapazität der Prozessor-Chipkarte nur ein kleiner Teil der medizinischen Daten eines Patienten berücksichtigt werden kann. So beinhaltet z.B. die Diabcard (GSF Forschungszentrum für Umwelt und Gesundheit Medis-Institut, Postfach 1129, D - 85758 Oberschleißheim) nur für die Diabetikerbetreuung relevante medizinische Daten. Bei einem anderen Modellprojekt, das von der Kassen-ärztlichen Vereinigung Koblenz, dem Zentralinstitut für die kassenärztliche Versorgung und der Bundesvereinigung Deutscher Apothekerverbände in den Städten Neuwied und Andernach gestartet wurde ("PraxisComputer", Nr. 4, 15. 06. 94, Seiten 3 bis 6, und Nr. 2, 10. 03. 95, Seiten 8/9), umfaßt der auf einer Patientenkarte gespeicherte Datensatz neben den Anamnesedaten nur noch den Impf- und den Röntgenstatus sowie vom Apotheker an Patienten abgegebene Medikamente.

Ein weiterer bedeutender Nachteil bestehender Speicherkarten besteht darin, daß die gespeicherten Daten nicht wirksam gegen Mißbrauch geschützt sind. Eine reine Verschlüsselung der gespeicherten Informationen ist nicht ausreichend, da mit einem ausreichend großen Aufwand letztendlich jeder Code entschlüsselt werden kann. Im Fall von Patientendaten bedeutet dies, daß letztere, beim Patienten selbst aufbewahrt, leicht auch Unbefugten in die Hände fallen können, die die entsprechenden Informationen nach dem Decodieren der Daten zum Nachteil des Patienten verwenden könnten.

Aus der DE 90 18 059 U1 ist ein System zur Speicherung, Bereitstellung und Aktualisierung von festen und/oder variablen Patienten- und Behandlungsdaten bekannt. Dabei wird von einer stationären Computer-Zentraleinheit mit einer Speichereinheit, in der die in einer Patientenkartei vorhandenen Patientendaten in Form von patientenspezifischen Datensätzen gespeichert sind, und einer Daten-Schnittstelle zur Ein- und Ausgabe von Patientendaten ausgegangen in Verbindung mit einer mobilen Einheit, wie etwa einem portablem Computer, der zur Aufnahme ausgewählter oder aller gespeicherten Patientendatensätze vorgesehen ist. So kann ein Arzt auch bei Hausbesuchen die Informationen seiner elektronischen Patientenkartei nutzen und letztere außer Haus direkt aktuell halten. Ein individueller Patientendatensatz kann über eine Krankenversichertenkarte aufgerufen und aktiviert werden, so daß die verwaltungstechnischen Besuchsdaten direkt erfaßt werden. Wieder zurück in seiner Praxis kann der Arzt dann die Daten auf seiner stationären Computer-Zentraleinheit durch die mobile Einheit aktualisieren.

Der Arzt nimmt hier also seinen eigenen Datensatz zum Patienten mit, anstatt vom Patienten selbst dessen gesammelte Daten von verschiedenen Ärzten und klinischen Einrichtungen zur Verfügung gestellt zu bekommen. Ärztliche Notdienste beispielsweise könnten dieses System bei unbekannten Patienten daher nicht nutzen. Es werden also von Anwendern und nicht von den Personen, welche die Daten betreffen, nur beschränkte Daten lediglich temporär transportabel abgespeichert.

In der DE 38 15 633 C2 ist eine Datenverarbeitungsanordnung zur zentralen Bearbeitung von medizinischen Daten offenbart. Hierbei werden transportable persönliche Speichereinrichtungen verwendet, die für eine vorübergehende Speicherung von laufend erfaßten Biodaten ausgelegt sind. Zumindest in vorgegebenen Zeitabständen werden daher die gespeicherten Daten über eine Telefonstrecke in die Datenverarbeitungsanlage eines Hospitals übermittelt. Sicherheitsaspekte spielen bei diesen Speichereinrichtungen daher keine Rolle.

Die beiden Druckschriften enthalten somit keine über übliche transportable Speichereinrichtungen hinausgehende Lösungen hinsichtlich der dezentralen Archivierung von persönlichen Daten.

Aber auch in anderen Lebensbereichen ist die Zusammenstellung, Handhabung und Verfügbarkeit von persönlichen Daten aufwendig und führt häufig nicht zum angestrebten vollumfänglichen Ergebnis. Neben der oftmals nicht ausreichenden Speicherkapazität der auf Prozessor-Chipkarten verfügbaren Datenspeicher für die gewünschte Datenmenge besteht bei einer Speicherung auf transportablen Massenspeichern die Gefahr einer mißbräuchlichen Verwendung auf Grund einer fehlenden Zugriffsabsicherung.

Aus WO-A-90 12464 ist ein Daten-Archivierungssystem mit zwei transportablen Speichervorrichtungen in Form des Speicherchips einer Chipkarte und einer CD-ROM bekannt. Diesen Speichereinrichtungen sind Berechtigungsprüfeinrichtungen zugeordnet, mittels denen nur in Abhängigkeit von einer positiven Berechtigungserkennung ein Zugriff auf Daten der CD-ROM möglich ist. Der Zugriff auf die CD-ROM soll mit Hilfe einer Vielzahl von Chipkarten möglich sein, so daß bei diesem vorbekannten System ein umfassender Datenschutz von vornherein ausgeschlossen ist. Um dennoch ein Mindestmaß an Datenschutz zu realisieren, benötigt die CD-ROM ein eigenes Inhaltsverzeichnis und eine eigene Sicherheitslogik, welche hardwaremäßig bei der Herstellung der CD-ROM in diese eingebracht werden müssen. Dies hat den Nachteil, daß sich die Herstellung dieser Speichervorrichtung verteuert und der Datenschutz mit hohem Aufwand verbunden ist.

Der Erfindung liegt die Aufgabe zugrunde, ein Daten-Archivierungssystem zu schaffen, das transportable Speichereinrichtungen zum Speichern und Aufbewahren von Daten enthält und einen höheren Sicherheitsstandard als bekannte Systeme ermöglicht.

Diese Aufgabe wird durch die Merkmalskombination des Anspruches 1 gelöst.

Erfindungsgemäß sind die erste und die zweite Speichervorrichtung und darauf gespeicherte Daten einem einzigen Inhaber zum Aufbewahren der Daten beim Inhaber zugeordnet. Nur in Verbindung mit der dem Inhaber zugeordneten ersten Speichervorrichtung kann auf die auf der zweiten Speichervorrichtung gespeicherten Daten zugegriffen werden. Dies ist eine besonders sichere Struktur, um einen unbefugten Zugriff auf die Informationen in der zweiten Speichervorrichtung auszuschließen. Diese Sicherheit wird dadurch erhöht, daß auf die auf der zweiten Speichervorrichtung gespeicherten Daten nur in Abhängigkeit von einer positiven Berechtigungs- und/oder Authentizitätserkennung mittels der Berechtigungsprüfeinrichtungen zugreifbar ist.

Durch die Einteilung der Speichereinrichtungen in einen ersten Teil und zumindest einen zweiten Teil ist eine Grobeinteilung der zu speichernden oder gespeicherten Daten möglich, wobei z. B. ein kleiner, schneller Speicher für häufig genutzte Daten und ein großer, langsamer Speicher für selten benötigte große Datenmengen mit Vorteil einsetzbar ist. Ferner kann ein Speicher für die Entscheidungsfindung der Berechtigungsprüfeinrichtungen erforderliche Informationen enthalten, wodurch auch der andere Speicher geschützt wird.

Insbesondere sind durch die Erfindung die Sicherheitsnachteile des Standes der Technik behoben. Ein Datenmißbrauch ist zumindest weitgehend unmöglich oder wenigstens in Anbetracht des erforderlichen Aufwandes unrentabel durchzuführen.

Dadurch, daß erfindungsgemäß ein Zugriff zumindest auf einige der Daten nur nach erfolgter Authentisierung und Autorisierung möglich ist, wird sichergestellt, daß die möglicherweise zusätzlich verschlüsselten Daten für Unbefugte nicht zugänglich sind, um diese Daten zur Informationsgewinnung zu decodieren.

Wenn ein solcher berechtigungsabhängiger Zugriff nicht für sämtliche Datenbereiche vorgesehen ist, können auch Daten gespeichert werden, die frei zugänglich sind, wie es beispielsweise für Notfalldaten von Vorteil sein kann.

Als weiterer wesentlicher Vorteil wird durch die Erfindung ferner die Möglichkeit geschaffen, größere persönliche Datenmengen dezentral mit ausreichender Sicherheit zu speichern, wofür transportable Speichereinrichtungen mit größeren Speicherkapazitäten in das System integriert werden können. Solange nämlich nicht die gewünschte Sicherheit der gespeicherten Daten vor unberechtigtem Zugriff realisierbar war, war die entsprechende Speicherung solcher bedeutenden persönlichen Daten im Zuständigkeitsbereich jeder Person selbst aus Datenschutzgesichtspunkten nicht vernünftig durchführbar. Erst durch die vorliegende Erfindung können nun auch große Datenmengen ausreichend geschützt werden, so daß nunmehr überhaupt erst sinnvoll an die Einbindung entsprechend ausreichend großer Speichermedien herangegangen werden kann, wie es beispielsweise durch bevorzugte Ausgestaltungen der Speichereinrichtungen der Erfindung erfolgt ist. D.h., daß es im speichertechnischen Sinn zwar grundsätzlich kein Problem darstellt, Massenspeicher für große Datenmengen bereitzustellen, derartige Speichermedien aber nicht selbst die für die persönlichen Daten erforderliche Zugriffssicherheit bieten, wie sie durch die Erfindung geschaffen wurde.

Beispielsweise kann der Zugang zu den gespeicherten Daten sowie der Umfang dieses Zugangs z.B. außer zu gespeicherten Notfalldaten ausschließlich vom Inhaber der Speichereinrichtungen bestimmt werden.

Die Sicherheitseinrichtungen bei dem erfindungsgemäßen Daten-Archivierungssystem, d.h. die Berechtigungsprüfeinrichtungen, können bei einer gegenseitigen Identifizierung von Speichereinrichtungen und Zugriffsgeräten beginnen und über die Registrierung einer Benutzererkennung durch die Berechtigungsprüfeinrichtungen bis zur Nachrichtenverschlüsselung und -authentifikation durch eine z.B. elektronische Unterschrift gehen.

Durch die im Erfindungsumfang enthaltenen Sicherheitsoptionen wird eine äußerst hohe Datensicherheit erreicht, wie sie beispielsweise nicht nur im medizinischen Bereich bislang noch nicht realisiert werden konnte.

Die Erfindung hat außerdem den Vorteil, daß z.B. EDV-Hardware verwendet werden kann, die bereits als Massenprodukt für einen günstigen Preis zur Verfügung steht, was den finanziellen Aufwand vernünftig abschätzen läßt und in vernünftigen niedrigen Grenzen hält. Durch entsprechende Schnittstellen mit gängigen Praxisverwaltungsprogrammen ist die Einbindung der vorgenannten üblichen Hardware oder einer speziellen Hardware und entsprechender Software in die vorhandene Praxis-EDV eines Arztes oder einer klinischen Einrichtung leicht möglich, was in der Bundesrepublik Deutschland z.B. über die vorhandene Schnittstelle "Behandlungsdatenträger" (BDT) gewährleistet ist.

Beispielsweise bei einer Anwendung der Erfindung als Patientendaten-Archivierungssystem kann eine direkte Personalisierung und Aktualisierung relevanter Daten in der Arztpraxis selbst erfolgen. Bereits jetzt ist absehbar, daß medizinische Daten zukünftig immer mehr primär auf z.B. digitalen Medien erfaßt und gespeichert werden. Bei dem erfindungsgemäßen System wird der Patient in die Lage versetzt, sich die Daten noch in der Arztpraxis direkt auf sein Speichermedium überspielen zu lassen, insbesondere medizinische Daten für Ausweisfunktionen und die Auflistung von Diagnosen und Dauermedikationen. Weitere umfangreichere Text- und Bilddaten können alternativ zur Überspielung auf die patientenspezifischen Speichereinrichtungen in der Arztpraxis in Personalisierungsbüros digitalisiert und auf die Speichereinrichtungen geschrieben werden. Eventuell kann vor einer Digitalisierung eine Verkleinerung oder sogar Mikroverfilmung durchgeführt werden, um den Speicherplatzbedarf zu minimieren.

Eine Alternative zu den persönlichen transportablen Speichereinrichtungen könnte in der multimedialen Datenkommunikation ein allgemeiner Datenverbund in der ambulanten Medizin sein, was beispielsweise mit Hilfe der ISDN-Vernetzung realisiert werden könnte. Die Telemedizin wird bereits in Krankenhäusern, Forschungsstätten und an Universitäten erprobt. Bei diesen Projekten geht es in erster Linie um Telekonsultation, also z.B. den Austausch von diagnostischem Bildmaterial zum Zweck der gemeinsamen Befundung. Daneben gibt es in diesem Bereich noch weitere sinnvolle Perspektiven, wie die Telekommunikation im administrativen Sektor oder die Teletherapie.

In der ambulanten Medizin sind aber völlig andere Voraussetzungen und Ziele gegeben. In der ambulanten Medizin kommt der Patient zum Arzt oder umgekehrt. Dabei kann der Patient problemlos als Überbringer von Daten fungieren, wodurch keinerlei Kosten entstehen, wie es im Vergleich mit der Datenvernetzung der Fall wäre. Bei einer Datenaustauschvernetzung besteht weiterhin die Gefahr, Produkte und Dienstleistungen am technisch Machbaren auszurichten und dabei die tatsächlichen Bedürfnisse des Patienten aus den Augen zu verlieren.

Bei der Telemedizin muß mit erheblichen Akzeptanzproblemen gerechnet werden. Der Austausch von persönlichen medizinischen Daten über Datenautobahnen schürt beim Patienten - vielleicht nicht zu Unrecht - die Angst vor dem "gläsernen Patienten". Ferner könnten bei einer Datenfernübertragung unautorisierte Personen eine Möglichkeit haben, an die gespeicherten Daten zu gelangen.

Es liegt aber eine weitere Nutzungsmöglichkeit der vorliegenden Erfindung darin, das Daten- oder insbesondere Patientendaten-Archivierungssystem und die Telemedizin synergetisch zu verbinden. So können die persönlichen Speichereinrichtungen, wie beispielsweise eine Prozessor-Chipkarte in Verbindung mit einem transportablen Massenspeicher unter Einbeziehung von geeigneten Zugriffsgeräten gleichsam als Peripheriestation dienen und patientenbezogene Daten nach erfolgter Schreibzugriffsfreigabe von verschiedenen Quellen aufnehmen. Oder in umgekehrter Richtung kann ein konsiliarisch hinzugezogener Arzt über z.B. das ISDN-Netz auf die Daten der Speichereinrichtungen zugreifen, sobald die Berechtigungserkennung erfolgt ist. Bei einer noch weiteren Anwendung kann ein Patient durch ein persönliches Zugriffsgerät bei einem Anruf bei einem Arzt beispielsweise parallel zum Gespräch über den zweiten Kanal des ISDN-Anschlusses dem Arzt seine Daten von den Speichereinrichtungen zur Verfügung stellen, wobei er mit seiner eigenen Berechtigung für die Zugriffsfreigabe sorgt.

Ein weiterer Vorteil der vorliegenden Erfindung liegt darin, daß bestehende Datensysteme und -strukturen problemlos übernommen und weitergeführt werden können. Z.B. kann bei einem Patientendaten-Archivierungssystem durch die Übernahme vorhandener Protokolle für einen Datenaustausch der in der Bundesrepublik Deutschland bereits eingeführten Krankenkassenkarte und des zugehörigen Lesegeräts ein frei zugänglicher Bereich der Speichereinrichtungen - vorzugsweise eine als eine Art Text- oder Textaufzeichnungskarte fungierende Prozessor-Chipkarte - im Empfangsbereich jeder Arztpraxis oder in der Aufnahmestation jedes Krankenhauses in Deutschland gelesen werden. Der Zugriff auf die Notfalldaten ist zumindest gegen einen Zugriff durch Ärzte nicht geschützt, so daß diese Daten auch bei Bewußtlosigkeit des Patienten zugänglich sind. Alternativ kann als gewisser Schutz der Notfalldaten eine Zugriffsgeräteidentifikation eingebaut sein, wobei Ärzte und Rettungsdienste dann zweckmäßigerweise über geeignete Zugriffsgeräte verfügen. Hierbei bezieht sich die Authentizitätsprüfung bzw. der Authentizitätsnachweis auf z.B. das Lesegerät selbst. Die Prozessorkarte, die ein Beispiel für einen geeigneten Speichereinrichtungsteil ist, ermittelt in diesem Fall also bedienerunabhängig, ob das jeweilige Lesegerät zugriffsberechtigt ist. Diese so geartete Prüfung kann auch für andere Datenbereiche als den Notfalldatenbereich verwendet werden.

Eine andere Möglichkeit, die Daten, die die Versorgung für den medizinischen Noffall betreffen in in einer Notsituation nicht hinderlicher Weise zu schützen, so daß sie nur einem bestimmten Personenkreis ohne großen Aufwand zugänglich sind, besteht in einer Arztkarte, durch die eine entsprechende Zugriffsfreigabe erreicht wird. Mit der Arztkarte oder allgemein einer "Profikarte" oder Berechtigungskarte kann sich eine per se zugriffsberechtigte Person gewissermaßen z.B. in ein Lesegerät einloggen. Bis zum Ausloggen der Person können über das Lesegerät die soweit freigegebenen Daten auf den Speichereinrichtungen gelesen werden. Somit kann personenbezogen die Zugriffsberechtigung medizinischer Leistungsbringer geregelt werden.

In jedem Fall (auch dem vorher geschilderten) wird auf der beispielsweise als ein Teil der Speichereinrichtungen vorgesehenen Prozessorkarte nach einem Zugriff vermerkt, mit welchem Lesegerät bzw. mit welcher Profikarte auf die Prozessorkarte zugegriffen wurde. So kann z.B. das Lesegerät eines bestimmten Krankenwagens ermittelt werden, über das ein Zugriff ausgeführt wurde. Über den Dienstplan kann dann der Name des verantwortlichen Arztes ermittelt werden. Verläuft eine Authentizitätsprüfung positiv, so erfolgt auch gleichzeitig die Autorisierung für einen Speicherzugriff durch z.B. die Prozessorkarte selbst.

Nur die sonstigen Daten z.B. der Textkarte und einer Bild- oder Bildaufzeichnungskarte als Massenspeicher, die durch einen Massenspeicher gebildet ist, können beispielsweise erst nach Eingabe einer nur dem Patienten bekannten, persönlichen Identifikationsnummer (PIN) abgerufen werden. Die Eingabe der PIN erfolgt durch den Patienten über eine separate Zahlentastatur im Sprechzimmer des Arztes. Auf z.B. der Prozessorkarte als einerseits Berechtigungseinrichtungsbestandteil und andererseits Speichereinrichtungsteil ist gewissermaßen ein Inhaltsverzeichnis der gesamten Speichereinrichtungen hinterlegt.

Aktuelle Untersuchungsbefunde, wie Labor- oder Blutdruckwerte, Daten für Vor- und Nachsorgeuntersuchungen, Pässe usw. können jederzeit vom behandelnden Arzt direkt in der Sprechstunde zur laufenden Dokumentation ergänzt werden. Ferner können z.B. Krankenkassen Updates versenden, die mittels eigenen Geräten, beim Arzt oder bei der Krankenkasse installiert werden können. Diese Daten können auf einem Massenspeicher beispielsweise in verschlüsselter Form vorliegen. Nur durch einen chipkarten-gestützten oder -freigegebenen Zugriff sind bei einem Ausführungsbeispiel diese Daten entschlüsselbar, wofür wegen der eventuell sehr großen Datenmenge ein Coprozessor als Kryptoeinheit eingesetzt wird, die z.B. ebenfalls auf der Chipkarte angeordnet ist.

Gemäß einer vorteilhaften Weiterbildung der Erfindung kann mittels den Berechtigungsprüfeinrichtungen eine Mehrzahl von Prüfstufen ausführbar sein. Der damit erzielbare Vorteil liegt darin, daß die gespeicherten Daten abgestuft vor unberechtigten Zugriffen geschützt werden können. So kann es beispielsweise wünschenswert sein, daß zumindest im eigenen Beisein ein Apotheker auf Informationen betreffend bisher verabreichte Medikamente zugreifen kann, ein Sachbearbeiter einer Bank bei einem Bankwechsel Dauerauftrags- und Einzugsermächtigungsdaten in die Datenbank des neuen Instituts überführt, ein Taxifahrer sich die exakt bezeichnete Zieladresse auf seinem Display anzeigen lassen kann, um nicht durch einen Verständigungsfehler zu einem falschen Ziel zu fahren oder das Ziel nicht zu kennen, usw.

Bei einer bevorzugten Ausgestaltung der Erfindung enthalten die Speichereinrichtungen zumindest teilweise ein zum Hinzufügen und/oder Ändern von Daten beschreibbares und insbesondere wiederbeschreibbares Speichermedium. Damit können in vorteilhafter Weise die gespeicherten Informationen problemlos aktuell gehalten werden, ohne bei Datenänderungen oder -hinzufügungen immer wieder neue komplette Speichereinrichtungen herstellen zu müssen, was im Hinblick auf eine möglichst große Sicherheit in Abhängigkeit von konkreten Anwendungsfällen aber auch Vorteile hat. Ein wiederbeschreibbares Speichermedium hat den Vorteil, daß der vorhandene Speicherplatz optimal genutzt werden kann, da keine unnötigen oder veralteten Daten gespeichert bleiben. Ein einmalig beschreibbares Speichermedium hat den Vorteil, daß Daten nicht versehentlich gelöscht werden und dabei unwiederbringbar verloren gehen können.

Dadurch, daß die Berechtigungsprüfeinrichtungen zum Freigeben eines Lese- und/oder Schreibzugriffs auf das beschreibbare Speichermedium in Abhängigkeit von einer insbesondere entsprechenden Berechtigungs- und/oder Authentizitätserkennung ggf. in einer zugehörigen Prüfstufe ausgelegt sind, kann vorteilhaft sichergestellt werden, daß die Zugriffsberechtigung möglichst praxisgerecht handhabbar ist. Dasselbe trifft für den Fall zu, daß zumindest ein Teil des beschreibbaren Speichermediums schreibgeschützt oder schreibschützbar ist, wie bei einer Weiterbildung der Erfindung vorgesehen ist.

Vorzugsweise enthalten die Speichereinrichtungen einen ersten Teil und zumindest einen zweiten Teil mit unterschiedlichen Speicherkapazitäten, wobei z.B. ein kleiner, schneller Speicher für häufig genutzte Daten und ein großer, langsamer Speicher für selten benötigte große Datenmengen mit Vorteil einsetzbar ist. Ferner kann ein Speicher für die Entscheidungsfindung der Berechtigungsprüfeinrichtungen erforderliche Informationen enthalten, wodurch auch der andere Speicher geschützt wird.

Bei einer bevorzugten Ausführungsform der Erfindung ist der erste Teil der Speichereinrichtungen kapazitätsmäßig am kleinsten und ein Ausweis-, Identifikations- oder Stammdatenspeicherteil. Praktischerweise kann gemäß einer Weiterbildung dieser Ausführung auf die auf dem ersten Teil der persönlichen Speichereinrichtungen gespeicherten Daten, wie Ausweis-, Identifikations- oder Stammdaten, unabhängig von den Berechtigungsprüfeinrichtungen oder mit einer positiven Berechtigungs- und/oder Authentizitätserkennung in einer ersten Prüfstufe der Berechtigungsprüfeinrichtungen zugegriffen werden. Damit steht ein Speicher zur Verfügung, der z.B. zum Speichern von Notfalldaten geeignet ist, die auch ohne Mitwirkung des Inhabers zugänglich sein sollten.

In weiterer Ausgestaltung ist bevorzugt, daß der zweite, relativ zum ersten Teil kapazitätsmäßig größere Teil der Speichereinrichtungen ein Detaildatenspeicherteil ist, dessen Inhalt somit besonders sicher ist. Diese Sicherheit wird dadurch erhöht, daß auf die auf dem zweiten Teil der persönlichen Speichereinrichtungen gespeicherten Daten, wie Detaildaten, nur in Abhängigkeit von einer positiven Berechtigungs- und/oder Authentizitätserkennung ggf. in einer zweiten Prüfstufe mittels der Berechtigungsprüfeinrichtungen zugreifbar ist.

Eine weitere besonders bevorzugte Realisierung der Erfindung besteht darin, daß separate erste bzw. zweite Speichervorrichtungen auf verschiedenen ersten bzw. zweiten Datenträgern angeordnet sind. Die räumliche Trennung der Speichereinrichtungsteile auf separaten Datenträgern trägt in vorteilhafter und besonders einfacher Weise zu einer hohen Sicherheit des erfindungsgemäßen Systems bei, da es dadurch möglich ist, den Datenträger mit den kritischeren Daten sicher aufzubewahren, solange er nicht benötigt wird, was meist relativ selten der Fall ist. Ferner ist das System so eingerichtet, wie weiter oben angegeben ist, daß auf den zweiten Speichereinrichtungsteil nur in Kombination mit dem ersten Speichereinrichtungsteil zugegriffen werden kann. Wird somit dafür gesorgt, daß nicht beide Speichereinrichtungsteile gemeinsam abhanden kommen können, sind die Daten auf dem zweiten Speichereinrichtungsteil absolut sicher.

Gemäß bevorzugten Ausführungen der Erfindung ist der erste Teil der Speichereinrichtungen bzw. die erste Speichervorrichtung eine magnetische, magneto-optische oder optische Speicherfläche oder ein Speicherchip insbesondere auf oder in einer vorzugsweise etwa scheckkartengroßen Kunststoffkarte als Datenträger. Ferner ist der zweite Teil der Speichereinrichtungen bzw. die zweite Speichervorrichtung bevorzugt ein elektronischer, magnetischer (z.B. Diskette oder Streamer-Tape), magneto-optischer oder optischer (z.B. CD-ROM ggf. beschreibbar oder WORM: "Write Once Read Many") Massenspeicher insbesondere in Form einer 2,5 oder 3,5 Zoll großen Diskette oder ein Halbleiterspeicher einer PCMCIA-Einheit (Personal Computer Memory Card International Association) als Datenträger. Als eine derartige Kombination können beispielsweise eine Prozessor-Chipkarte mit den Berechtigungsprüfeinrichtungen und einem kleineren Speicher und eine MO- (magneto-optische) Platte zur Speicherung großer Datenmengen einschließlich Bildern kombiniert eingesetzt werden, wobei der Zugriffsschutz der MO-Platte durch die Prozessor-Chipkarte erfolgt, für einen Zugriff auf die MO-Platte also zumindest die Prozessor-Chipkarte vorhanden sein muß.

Die Verteilung z.B. medizinischer Informationen auf beispielsweise eine Prozessor-Chipkarte und ein Massenspeichermedium gewährleistet einen per se abgestuften Datenzugang und somit eine hohe Sicherheit, die weiterhin dadurch erhöht wird, daß die Chipkarte gewissermaßen als Schlüssel einerseits für einen Zugriff auf die auf ihr selbst gespeicherten Daten und andererseits auf das Massenspeichermedium fungiert. Zusätzlich können auch noch die Daten auf dem Massenspeichermedium verschlüsselt sein, um eine noch höhere Sicherheit zu gewährleisten.

Als Prozessor-Chipkarte, die bei der vorstehend beschriebenen Kombination mit einem Massenspeicher als "Textkarte" fungiert, kann eine Prozessorkarte entsprechend den geltenden DIN-Vorschriften oder ISO-Standards mit einer Speicherkapazität von acht oder sechszehn Kilobytes verwendet werden, was bei einer Komprimierung der darauf zu speichernden Daten eine ausreichende Informationsmenge für Notfälle und Übersichten ermöglicht. Der Massenspeicher, der auch als "Bildspeicher" bezeichnet werden kann, hat als Minidisk oder magneto-opische Platte (MO-Platte) beispielsweise eine Kapazität von 140 Megabyte oder sogar bis zu 650 Megabyte. MO-Platten sind mit 2,3 oder 3,5 Zoll relativ klein und handlich und können ausreichend große Datenmengen aufnehmen. Hinzu kommt, daß die Daten auf einer MO-Platte beliebig oft überschrieben, aber unter Alltagsbedingungen nicht versehentlich gelöscht werden können.

Die Textkarte speichert in Schriftform vorliegende Informationen und leistet durch eigene "Intelligenz" den Zugriffsschutz für beide Datenträger beispielsweise unter Einbeziehung von Verschlüsselungsalgorithmen, während die Bildkarte die Speicherung von großen Datenmengen übernimmt, also von Abbildungen (Röntgen-, Ultraschall- und Endoskopiebilder usw.), von Biosignalen (z.B. EKG, EEG) oder auch von Texten zur Anamnese, die auf der Prozessorkarte keinen Platz mehr finden. Insbesondere kann die Textkarte bei einem Patientendaten-Archivierungssystem Informationen, wie Verwaltungsdaten, einen Noffall-Ausweis, Anamnese, Befund, Vor- und Nachsorge sowie einen Paß u.ä. enthalten.

Für die Erfindung kommt es jedoch nicht darauf an, ob und ggf. wie die Speichermedien zusammengesetzt sind, sondern nur darauf, daß ein wirksamer Zugriffsschutz realisiert ist. Beispielsweise kann ein optischer Speicher mit sehr großer Kapazität auch als einziger Speicher verwendet und auf einer etwa scheckkartengroßen Kunststoffkarte untergebracht werden. Durch physikalisches Lochbrennen sind beispielsweise Speicherkapazitäten im Bereich von 1 Gigabyte pro cm² Kunststoffolie als Datenträger möglich, so daß bezüglich einer so ausgestalteten Karte externe Massenspeicher unnötig . sind.

Ein vorzugsweiser Aufbau der Erfindung besteht darin, daß zumindest einem Teil der Speichereinrichtungen Prozessoreinrichtungen zugeordnet sind, die vorzugsweise auf dem Datenträger, wie z.B. einer Kunststoffkarte, angeordnet sind und insbesondere einen Prozessor enthalten. Besonders vorteilhaft können derartige Prozessoreinrichtungen für die Berechtigungsprüfeinrichtungen genutzt werden. Die Prozessoreinrichtungen gewährleisten dabei durch eine eigene "Intelligenz" den Zugriffsschutz für die Speichereinrichtungen. Dabei ist weiterhin bevorzugt, daß die Prozessoreinrichtungen zur Steuerung von zumindest Teilen der Speichereinrichtungen und/oder zur wenigstens teilweisen Steuerung von Zugriffen auf letztere und/oder insbesondere als Teil der Berechtigungsprüfeinrichtungen ausgelegt sind.

Zur Erhöhung der Datensicherheit ist es ferner von Vorteil, wenn den Speichereinrichtungen Kryptoeinrichtungen vorgeschaltet sind, deren Betrieb insbesondere mittels der Berechtigungsprüfeinrichtungen zur Bearbeitung von Zugriffen auf die Speichereinrichtungen freigebbar ist. D.h., daß ein Ver- und Entschlüsseln der gespeicherten Daten von einer Zugriffsfreigabe durch die Berechtigungseinrichtungen abhängt. Bevorzugt sind die Kryptoeinrichtungen in ggf. vorhandenen Prozessoreinrichtungen enthalten und weisen vorzugsweise einen Kryptoprozessor auf.

Wenn ein Teil der Speichereinrichtungen und/oder die Berechtigungsprüfeinrichtungen auf einer Prozessor-Chipkarte realisiert sind, kann beispielsweise letztere auch eine Kryptoeinheit aufnehmen. Prozessoreinrichtungen können aber auch ganz oder teilweise in einem Zugriffsgerät oder in zugeordneten Rechnereinrichtungen untergebracht sein.

Allgemein können Kryptoeinrichtungen z.B. ein asymmetrisches Verschlüsselungsverfahren (RSA) verwenden.

Um zu verhindern, daß unerwünschte Kopien der gespeicherten Daten angefertigt werden können, sieht eine Fortbildung der Erfindung vor, daß Kopierschutzeinrichtungen vorgesehen sind, mittels denen ein Speichern von zumindest einigen Daten, die auf den persönlichen Speichereinrichtungen, insbesondere ggf. deren zweiten Teil, gespeichert sind, auf bezüglich letzteren externen Speichern unterbindbar ist und/oder ein Speichern von zumindest einigen Daten, die auf dem ersten Teil der Speichereinrichtungen gespeichert sind, auf bezüglich letzteren externen Speichern zulaßbar sind. Letzteres ermöglicht z.B. die problemlose Übernahme von Patientenname, -adresse und Krankenkassendaten für die Rezeptausstellung und Behandlungsabrechnung.

In ähnlicher Weise wirken Druckausgabeschutzeinrichtungen, die vorgesehen sein können und mittels denen eine Druckausgabe von zumindest einigen Daten unterbindbar ist, die auf den persönlichen Speichereinrichtungen, insbesondere ggf. deren zweiten Teil, gespeichert sind und/oder ein Drucken von zumindest einigen Daten zulaßbar ist, die auf dem ersten Teil der Speichereinrichtungen gespeichert sind. Letzteres ermöglicht eine problemlose Erstellung z.B. eines schriftlichen Behandlungsberichts für einen Arzt.

Um zu verhindern, daß die einer Person genehmigte Einsichtnahme in die Daten durch Hardware-Einrichtungen gleichzeitig unbeabsichtigt beispielsweise über zusätzliche Bildschirme oder mittels Netzwerkkarten sogar andere Rechner auch weiteren Personen zugänglich sind, sollten Ausgabebeschränkungseinrichtungen vorgesehen sein, mittels denen eine Ausgabe von zumindest einigen Daten, die auf den persönlichen Speichereinrichtungen, insbesondere ggf. deren zweiten Teil, gespeichert sind, auf mehr als ein Ausgabemedium unterbindbar ist.

Um jedoch willentliche Kopien, Druckausgaben und Mehrbildschirmanzeigen, Netzwerkübertragungen etc. der aufgerufenen Daten zuzulassen, können die entsprechenden vorgenannten Schutzeinrichtungen mittels der Berechtigungsprüfeinrichtungen durch eine entsprechende positive Berechtigungs- und/oder Authentizitätserkennung ggf. in einer zugehörigen Prüfstufe neutralisiert werden.

Eine einfache, schnelle und wirkungsvolle Sicherungseinrichtung besteht darin, daß die Berechtigungsprüfeinrichtungen ausgelegt sind, Gerätekennungen von Zugriffsgeräten für die Handhabung der Speichereinrichtungen in eine Prüfung zur Berechtigungs- und/oder Authentizitätserkennung ggf. zur Erfüllung einer insbesondere ersten Prüfstufe einzubeziehen. Eine derartige Berechtigungsprüfung kann z.B. für eine Zugriffsfreigabe auf Notfalldaten ausreichend sein und durch Bereitstellen entsprechender Zugriffsgeräte für Notdienstpersonal oder -fahrzeuge sicher realisiert werden.

Zusätzlich oder alternativ können die persönlichen Speichereinrichtungen Kennungen aufweisen, die von den Berechtigungsprüfeinrichtungen in eine Prüfung zur Berechtigungs- und/oder Authentizitätserkennung ggf. zur Erfüllung einer insbesondere ersten Prüfstufe einbeziehbar sind. Damit kann beispielsweise sichergestellt werden, daß es sich nicht um gefälschte Speichereinrichtungen handelt, deren falsche Daten im medizinischen Bereich zu einer gefährlichen Falschbehandlung führen können. Auch daran ist deutlich zu sehen, wie wichtig z.B. im Bereich der Patientendatenarchivierung die zuverlässige Bereitstellung der richtigen Daten ist.

Die bei der vorliegenden Erfindung bevorzugt eingesetzten Sicherheitskomponenten sind Benutzer- und/oder Inhaberidentifikationen von zumindest einem Anwender bzw. dem Inhaber, dessen Daten in den Speichereinrichtungen archiviert sind. Derartige Kennungen werden von den Berechtigungsprüfeinrichtungen vorzugsweise in eine Prüfung zur Berechtigungs- und/oder Authentizitätserkennung ggf. zur Erfüllung einer insbesondere zweiten Prüfstufe und ggf. weiterer Prüfstufen zur Freigabe eines Zugriffs auf zumindest einige der auf den persönlichen Speichereinrichtungen gespeicherten Daten einbezogen.

Besonders einfache Möglichkeiten zur Eingabe von Anwenderkennungen, wie eine Benutzer- bzw. Inhaberidentifikation, für die Berechtigungsprüfeinrichtungen erfolgt über manuelle und/oder elektronische Eingaben, wie beispielsweise von einer Berechtigungskarte.

Um jederzeit vorgenommene Informationszugriffe und Einträge personell zuordnen zu können, ist es vorteilhaft, wenn durch die Berechtigungsprüfeinrichtungen zugelassene Zugriffe auf den Speichereinrichtungen mit den dafür relevanten Daten, wie Gerätekennungen von Zugriffsgeräten und/oder Benutzer- und/oder Inhaberidentifikationen, insbesondere auf den Speichereinrichtungen selbst dokumentierbar sind.

Um dem Inhaber der Speichereinrichtungen die darin oder darauf enthaltenen Informationen zugänglich zu machen, oder um ihm selbst Aktualisierungen zumindest bestimmter Daten zu ermöglichen, sind bei einer vorzugsweisen Ausgestaltung des erfindungsgemäßen Systems persönliche Zugriffsgeräte vorgesehen, mittels denen vom Inhaber, dessen Daten in den Speichereinrichtungen archiviert sind, und/oder von Anwendern der Speichereinrichtungen in Abhängigkeit von einer insbesondere entsprechenden Berechtigungs- und/oder Authentizitätserkennung ggf. in einer zugehörigen Prüfstufe auf zumindest einige der archivierten Daten zum Zweck der Information, Datenänderung/-ergänzung und/oder Weitergabe auch über Datenfernübertragung zugreifbar ist. Damit können z.B. auch bei telefonischen Beratungen, Vereinbarungen, Bestellungen etc. erforderliche Daten problemlos dem Gesprächspartner zur Verfügung gestellt werden. Eine derartige Datenfernübertragung wird z.B. durch das zweikanalige ISDN-Netz begünstigt, in dem ohne weiteres die Gesprächs- und die Datenübertragung gleichzeitig möglich sind.

Für Kunden-, Patienten- und Mandantenbesuche einerseits und die Arbeit in der Firma, Praxis bzw. Kanzlei andererseits ist es weiter von Vorteil, wenn mobile und/oder stationäre Zugriffsgeräte vorgesehen sind, mittels denen von Anwendern in Abhängigkeit von einer insbesondere entsprechenden Berechtigungs- und/oder Authentizitätserkennung ggf. in einer zugehörigen Prüfstufe auf zumindest einige der archivierten Daten zum Zweck der Information und/oder Datenänderung/-ergänzung zugreifbar ist. Dadurch kann das System überall und jederzeit einsatzbereit gehalten und können z.B. auch Rettungsfahrzeuge, Polizeifahrzeuge usw. problemlos so ausgestattet werden, daß eine Nutzung der gespeicherten Informationen an jeglichem Einsatzort möglich ist.

Für die Kosten der Zugriffsgeräte ist es von Vorteil, daß letztere beispielsweise grundsätzlich für einen Schreib- und/oder Lesezugriff ausgelegt sind, wobei zumindest auf einige der auf den persönlichen Speichereinrichtungen gespeicherten Daten eben in Abhängigkeit von einer insbesondere entsprechenden Berechtigungs- und/oder Authentizitätserkennung ggf. in einer zugehörigen Prüfstufe zum Schreiben und/oder Lesen zugegriffen werden kann.

Für die bereits weiter oben angegebene besonders einfache Berechtigungsüberprüfung ist vorgesehen, daß die Zugriffsgeräte Gerätekennungen aufweisen, die von den Berechtigungsprüfeinrichtungen zur Prüfung einer Berechtigungs- und/oder Authentizitätserkennung ggf. zur Erfüllung einer insbesondere ersten Prüfstufe erfaßbar sind, und daß die Gerätekennung vorzugsweise durch eine in zumindest ein Zugriffsgerät eingebbare Anwenderkennung aktivierbar ist.

Das System kann besonders kostengünstig und einfach aufgebaut werden, wenn zum Ansteuern der Zugriffsgeräte herkömmliche Rechnereinrichtungen, wie PCs, vorgesehen sind. Weiterhin wird ein günstiger Aufbau des erfindungsgemäßen Daten-Archivierungssystems dadurch gefördert, daß die Zugriffsgeräte entsprechend den Formaten von die Speichereinrichtungen aufnehmenden Datenträgern Kartenlesegeräte für z.B. Magnetstreifen- oder Chipkarten, Diskettenlaufwerke für z.B. magnetische, magneto-optische oder optische Disketten, CD-ROM-Laufwerke und/oder Aufnahmen für PCMCIA-Einheiten enthalten, wie sie insbesondere weitverbreitet angeboten werden. Ein Sicherheitsrisiko entsteht dadurch nicht, da die Datensicherheit durch die Berechtigungsprüfeinrichtungen gewährleistet wird. Ferner können herkömmliche Rechnereinrichtungen, einschließlich wenigstens Bildschirm, Tastatur und Drucker, kostengünstig als Anzeige- und Ein-/Ausgabegeräte von Daten vorgesehen werden.

Eine besonders effiziente, sichere und zuverlässige Ausgestaltung der Zugriffsgeräte sowie ein entsprechender Betrieb des Systems nach der Erfindung besteht darin, daß zumindest zwei Zugriffsgeräte zur Zusammenschaltung von entsprechenden Datenträgern der Speichereinrichtungen insbesondere zu einem Gesamtgerät kombiniert sind und diese Zusammenschaltung von den Berechtigungsprüfeinrichtungen zum Berechtigungsnachweis oder ggf. als eine erste Prüfstufe erfaßbar ist. Beispielsweise kann ein kombiniertes Zugriffsgerät für eine Prozessor-Chipkarte und einen Massenspeicher als Bestandteile der Speichereinrichtungen vorgesehen werden. Die Berechtigungsprüfeinrichtungen können hierbei besonders einfach realisiert werden, da die Schaltung fest vorgegeben sein kann. Insbesondere können die Sicherheitseinrichtungen in Form der Berechtigungsprüfeinrichtungen unabhängig vom Betriebssystem eines zur Steuerung, Bedienung und Auswertung verwendeten Rechners betrieben werden. Vor allem bei dieser Ausführungsform, aber grundsätzlich auch jeder anderen geeigneten Gestaltung der Erfindung, kann besonders einfach und effizient eine Zugriffsgerätekennung zum Datenschutz eingesetzt werden.

Zunächst kann dabei eine Autorisierung des Benutzers erfolgen. Es wird nur dann ein Zugriff auf das Speichermedium erlaubt, wenn das verwendete Zugriffsgerät, das ein geeignetes Laufwerk sein kann, insbesondere für zwei Datenträger, wie z.B. eine Prozessor-Chipkarte und eine Minidisk, mit einer Kennung ausgestattet ist. Dadurch wird gewährleistet, daß nur entsprechend ausgerüstete Zugriffsgeräte, die auch nur für einzelne Datenträger ausgelegt sein können, für Schreib- und/oder Leseoperationen benutzt werden können, was sicherstellt, daß nur dafür autorisierte Einrichtungen Zugang zu den Daten der Speichereinrichtungen erhalten.

Bei einem Ausführungsbeispiel der Erfindung haben zur Datensicherung sowohl das Lesegerät einer Prozessor-Chipkarte als auch das Laufwerk einer Diskette je eine Kennung. Dabei ist die Diskette nur lesbar, wenn die Lesegerätekennung die Prozessorkarte autorisiert und gleichzeitig die Laufwerkskennung die Diskette autorisiert und gleichzeitig die Prozessorkarte die Diskette freigibt.

Ferner kann bereits über die Zugriffsgerätekennung jeglicher Zugriff auf die Daten der Speichereinrichtungen dokumentiert werden. Dies würde in der Weise erfolgen, daß bei Verwendung des Zugriffsgeräts, wie eines einzelnen oder für mehrere Speichervorrichtungen kombinierten Laufwerks, die entsprechende Kennung als Stempel an einem dafür vorgesehenen Speicherplatz z.B. auf der Prozessor-Chipkarte als Teil der Speichereinrichtungen hinterlassen wird.

Schließlich ist über die aufgezeichnete Zugriffsgerätekennung eine Identifikation des Benutzers möglich. Damit kann nachgewiesen werden, in welcher Arztpraxis oder in welchem Rettungswagen auf die Daten der Speichereinrichtungen zugegriffen wurde. Durch entsprechende Maßnahmen, wie z.B. turnusmäßiger Wechsel der Kennung, kann damit auch für die Authentifikation des Benutzers gesorgt werden.

Damit ein sicherheitstechnisch zwar nicht zu fürchtender, informationstechnisch jedoch äußerst unangenehmer Datenträgerverlust sich nicht negativ auf die Datenarchivierung auswirkt, sollten von allen Datenträgern Sicherungskopien erstellt und aufbewahrt werden. Bevorzugt sind die Zugriffsgeräte ausgelegt, ohne Berechtigungsnachweis oder ggf. in einer ersten Prüfstufe Sicherungskopien der transportablen, persönlichen Speichereinrichtungen zu erstellen. Besondere Sicherheitsmaßnahmen sind dabei deshalb nicht zu beachten, da für den Zugriff auf Kopien der Speichereinrichtungen dieselben Maßgaben gelten, wie für die Originale.

Für einen bevorzugten Einsatz des erfindungsgemäßen Daten-Archivierungssystem sind gewisse Anpassungen an die Handhabung von Patientendaten vorteilhaft, wie z.B., daß das System zum Speichern und Aufbewahren von Patientendaten, einschließlich schriftlicher und graphischer Unterlagen, beim Inhaber ausgelegt ist. Dafür kann das System geeignete Eingabemedien, wie einen Scanner enthalten.

Vorzugsweise enthält der erste Teil der Speichereinrichtungen einen Notfalldatenspeicherbereich, in dem Verwaltungsdaten und die Daten eines Notfallausweises, eines Organspendeausweises, Notfalltestaments u.ä. des Inhabers speicherbar sind. Ein Zugriff auf diese Daten sollte durch die Berechtigungsprüfeinrichtungen frei oder mit nur einer Arzt- oder Rettungsdienstkennung, die vorzugsweise über eine spezielle Arzt- oder Rettungsdienstkarte oder eine Arzt-/Rettungsdienst-Zugriffsgerätekennung in die Berechtigungsprüfeinrichtungen eingebbar ist, oder mit nur einer Inhaberkennung freigebbar sein.

Weiterhin ist es bevorzugt, daß der erste Teil der Speichereinrichtungen einen Übersichtsdatenspeicherbereich enthält, in dem eine Auflistung wichtiger anamnestischer Daten, einschließlich einer ggf. erfolgten Verabreichung von Blut oder Blutprodukten und eine Dokumentation wichtiger Gesundheits-/Krankheitsdaten, einschließlich Allergie-, Impf-, Röntgen-, Schrittmacher-, Diabetiker-, Medikamentendaten u.ä., analog einer ärztlichen Patientenkarte gespeichert werden kann. Zusätzlich zu den vorstehenden im Zusammenhang mit den Notfalldaten angegebenen Zugriffsberechtigungen kann hier noch ein Zugriff durch Krankenkassen mittels einer entsprechenden Kennung vorgesehen sein.

Alle diese Zugriffsrechte können ferner für einen im ersten Teil der Speichereinrichtungen enthaltenen Behandlungsdatenspeicherbereich, in dem ein vorgegebener zeitlicher Rahmen und eine vorgegebene inhaltliche Struktur von Vorsorgeuntersuchungen sowie Untersuchungen im Rahmen der Nachsorge chronischer und/oder bösartiger Erkrankungen speicherbar sind, und für einen optionalen oder zusätzlichen Nachweisdatenspeicherbereich eingestellt sein, der im ersten Teil der Speichereinrichtungen enthalten ist und in dem zumindest eine vorgebbare Anzahl und/oder Art von Zugriffen auf die Speichereinrichtungen insbesondere einschließlich der jeweils erfolgten vorzugsweise individuellen Berechtigungs- und/oder Authentizitätserkennung und vorgenommenen Zugriffsart im einzelnen speicherbar ist.

Eine vorzugsweise Gestaltung des ersten Teils der Speichereinrichtungen kann einen Dokumentationsbereich enthalten, in dem die im Laufe des Lebens des Inhabers der Speichereinrichtungen anfallenden relevanten Gesundheits-/Krankheitsdaten einschließlich Anamnese, Diagnosen, Befunde wie Labor-, Blutdruckwerte, etc. speicherbar sind.

Eine vorzugsweise Gestaltung des zweiten Teils der Speichereinrichtungen kann einen Dokumentationsbereich enthalten, in dem alle Originalunterlagen von Arztberichten, Zeugnissen, Röntgen-, Ultraschall-, Computertomographie-, Endoskopieabbildungen usw., Biosignale, wie EKG- und EEG-Daten etc., direkt oder als Graphiken u.ä. speicherbar sind.

Ein Zugriff auf die vorgenannten Dokumentationsbereiche ist zumindest soweit durch die Berechtigungsprüfeinrichtungen einzeln oder in Kombination mit einer Arztkennung, welche vorzugsweise über eine spezielle Arztkarte oder eine Arzt-Zugriffsgerätekennung in die Berechtigungsprüfeinrichtungen eingebbar ist, mit einer Inhaberkennung und/oder einer Krankenkassenkennung freigebbar.

Auch der zweite Teil der Speichereinrichtungen kann bei einer bevorzugten Ausgestaltung einen Nachweisdatenspeicherbereich enthalten, der grundsätzlich genauso funktioniert, wie der Nachweisdatenspeicherbereich im ersten Teil der Speichereinrichtungen. Eine vorteilhafte Weiterbildung davon besteht darin, daß der Nachweisdatenspeicherbereich im ersten Teil der Speichereinrichtungen schieberegisterartig aufgebaut ist, so daß sein ältester Inhalt zur insbesondere endgültigen Abspeicherung in den Nachweisdatenspeicherbereich des zweiten Teils der Speichereinrichtungen zur Platzschaffung für einen Neueintrag im Nachweisdatenspeicherbereich im ersten Teil der Speichereinrichtungen verschiebbar ist. Damit können die Nachweisdaten jederzeit gespeichert werden. Es kann insbesondere auch bei anderen Einsatzgebieten des persönlichen Archivierungssystems nach der Erfindung vorgesehen werden, daß nur die letzten etwa drei bis fünf Zugriffsinformationen gespeichert bleiben und ältere Zugriffsinformationen gelöscht werden.

Vorteilhafterweise kann eine bereits bestehende und bei der Bevölkerung eingeführte Karte, wie eine Krankenversicherungskarte, ein Personalausweis oder eine Kontokarte, die Speichereinrichtungen des erfindungsgemäßen Daten-Archivierungssystems ganz oder teilweise aufnehmen.

Für eine weitere Erhöhung der Sicherheit der archivierten Daten ist es bevorzugt, daß mittels den Berechtigungsprüfeinrichtungen ein insbesondere turnusmäßiger Kennungswechsel einer Anwender- und/oder Gerätekennung zur Berechtigungs- und/oder Authentizitätserkennung berücksichtigt werden kann. Dabei wird vorzugsweise mittels den Berechtigungsprüfeinrichtungen eine Authentizitäts- und/oder Berechtigungsprüfung von Anwender, Zugriffsgeräten und/oder Speichereinrichtungen für einen beabsichtigten Zugriff durchgeführt.

Die Verwendungsmöglichkeit der Daten im Daten-Archivierungssystem nach der Erfindung wird weiter dadurch optimiert, daß die Speichereinrichtungen eine Mehrzahl von Bereichen enthalten, denen unterschiedliche Zugriffssicherungen, wie Passwörter, PINs o.ä. zugeordnet werden können.

Ein besonderer weiterer Schutz kann bei der Erfindung vorteilhaft für Schreibzugriffe vorgesehen sein, indem Speicherungen von neuen oder geänderten Daten auf die Speichereinrichtungen dort nur nach Eingabe einer Signatur zusammen mit dieser abspeicherbar sind, und daß letztere vorzugsweise durch eine Anwender- und/oder Zugriffsgerätekennung insbesondere automatisch erzeugt wird.

Nachfolgend werden einige weitere bevorzugte Ausführungsformen angegeben, die sich aus verschiedenen Merkmalskombinationen der Unteransprüche ergeben.

Beispielsweise als Patientenkarte kann eine Prozessor-Chipkarte Verwendung finden, die sowohl zumindest einen Teil der Berechtigungsprüfeinrichtungen in Form des Prozessors als auch einen ersten Teil der Speichereinrichtungen auf dem Chip enthält, um ein Patientendaten-Archivierungssystem zu realisieren. Mit einer derartigen Prozessor-Chipkarte kann der Informationsaustausch im Medizinbereich verbessert werden. In erster Linie dient die Prozessor-Chipkarte dabei als Kontroll- und Sicherheitselement, und die Speicherfunktion ist insbesondere auf Grund der üblicherweise geringen Speicherkapazität solcher Karten zweitrangig. Das erfindungsgemäße System enthält daher neben der Karte einen mobilen eigentlichen Datenspeicher, wofür sich beispielsweise eine magneto-optische Platte in Form einer 2,5-Zoll-Diskette eignet. Diese Datenträger sind so klein und handlich wie eine Chipkarte, verfügen aber über eine Speicherkapazität von beispielsweise 140 Megabyte und sind wiederbeschreibbar.

Die durch die Erfindung geschaffene Sicherheit wird technologisch durch die Kombination der Prozessor-Chipkarte mit einem bezüglich dieser externen Massendatenspeicher erreicht. Der Zugriff zu dem Massendatenspeicher erfolgt ausschließlich über die Prozessor-Chipkarte mit ihren Sicherheitseinrichtungen zur Berechtigungsprüfung und Zugriffsfreigabe. Damit sind die Daten z.B. auf der magneto-optischen Platte in gleicher Weise geschützt, als ob sie auf der Prozessor-Chipkarte selbst abgelegt wären.

Somit hat ein Patient in dieser Zeit der zunehmenden medizinischen Aufsplittung mehr als nur einen medizinischen Ausweis und wird in die Lage versetzt, seine relevanten Krankheitsdaten selbst komplett zu dokumentieren, um sie dann beispielsweise dem jeweils behandelnden Arzt oder seiner Versicherung zur Verfügung stellen zu können. Die durch die Erfindung somit allgemein mögliche umfassende persönliche und insbesondere medizinische Datensammlung in der Hand des Patienten trägt zu einer Verbesserung des Informationsaustausches z.B. zwischen den Ärzten bei. Gleichzeitig ist sie, um beim Beispiel des Patientendaten-Archivierungssystems zu bleiben, ein Instrument zur Qualitätssicherung der ärztlichen Leistung und dient einer ganzheitlichen Betreuung des Patienten. Die Vermeidung von Datenverlusten z.B. bei einem Arztwechsel führt zu einer Reduzierung der erforderlichen Untersuchungen und damit zu einer Entlastung, gesundheitsmäßig beim Patienten und kostenmäßig bei den Krankenkassen.

Eine Datensammlung über die Krankengeschichte darf sich aber nicht in einer Auflistung von anamnestischen Daten, Diagnosen oder durchgeführten Untersuchungen erschöpfen. Jeder weiterbehandelnde Arzt möchte zumindest die kompletten Arzt- und Krankenhausberichte einsehen, und häufig will er verständlicherweise auch nicht darauf verzichten, die Originalaufzeichnungen und -befunde betrachten zu können. Eine solche umfassende Datensammlung wäre aber auf einer herkömmlichen Prozessor-Chipkarte alleine nicht realisierbar, sondern nur in Kombination mit einem typischen Datenspeicher, für den es ohne die Erfindung keinen ausreichenden Datenschutz gäbe.

In Fortführung des vorbeschriebenen Ausführungsbeispiels kann die Prozessor-Chipkarte zunächst als Noffall-Ausweis fungieren. Neben den typischen Notfalldaten kann der entsprechende Speicherteil bei Interesse auch als zum Speichern eines Organspendeausweises oder Notfalltestaments eingesetzt werden. Mögliche Zugriffsregelungen hierfür wurden bereits weiter oben beschrieben. Durch die Mitwirkung des Inhabers über einen Zugriffsschlüssel, wie ein Passwort oder eine PIN wird daraus eine umfassende Ausweiskarte mit den wesentlichen medizinischen (oder bei anderen Anwendungsfällen Finanz-, Versicherungs-, "Kleingeldguthaben-") Basisdaten. Zusammen mit dem z.B. elektronischen Massenspeichermedium für die Komplettdaten erhält der Patient eine vollständige Sammlung von wichtigen Krankheitsdaten, wobei die Arztberichte, Bilder (beispielsweise Röntgen- und Ultraschallbilder) und Biosignale (EKG u.a.) im Original vorliegen. Aus praktischen Überlegungen kann die Mitwirkung des Patienten bei der Zugriffsfreigabe für einen Arzt, der sich z.B. neben seinem Kartenlesegerät und seinem Laufwerk für die Berechtigungsprüfeinrichtungen beispielsweise mit einer Arztkarte identifiziert hat, darauf beschränkt werden, daß auf der Prozessor-Chipkarte ein Foto des Patienten den Karteninhaber ausweist.

Es kann ferner eine automatisch arbeitende Einrichtung vorgesehen sein, die dafür sorgt, daß der Speicher einer gefüllten Prozessor-Chipkarte durch Auslagern von Daten auf z.B. eine zugehörige MO-Platte wieder zum Aufnehmen weiterer neuer Daten bereit ist.

Weiterhin ist bei der Ausführung mit der Prozessor-Chipkarte als Kontroll- und Sicherheitsinstrument eine Ausstattung mit einem Coprozessor möglich, durch den für das Sicherheitskonzept eine Authentifikation von Terminal, Karte und Benutzer sowie eine Dokumentation über jeden Zugriff auf die abgelegten Daten erfolgen kann.

Als Berechtigungsprüfeinrichtungen und deren Prüfmittel sind insbesondere Prozessoreinrichtungen vorzugsweise auf einem Teil der oder allgemein den Speichereinrichtungen und/oder aber in einem Steuerrechner oder Zugriffsgerät und geeignete Programme bzw. Zugriffsgeräte-, Speichereinrichtungs-, Inhaber- und Anwenderkennungen zu nennen. Die Sicherung kann beispielsweise ferner auch oder zusätzlich dadurch erreicht werden, daß die Kennungen getrennter Speichervorrichtungen und insbesondere Datenträger kombiniert werden müssen, um auf geschützte Daten zugreifen zu können. Ferner können die Daten direkt in lauffähigen Programmdateien enthalten sein, deren Aufruf von bestimmten Kennungen abhängt. Der erfindungsgemäße Grundschutz wird aber dadurch erreicht, daß ein Zugriff vom Prüfergebnis der Berechtigungsprüfeinrichtungen abhängt.

Das erfindungsgemäße System kann zwar z.B. eine Arztkartei schon wegen der bestehenden Dokumentationspflicht nicht ersetzen. Die Erfindung schaffl aber eine wichtige Ergänzungsmöglichkeit beispielsweise zur Arztkartei, da durch das Patientendaten-Archivierungssystem sämtliche relevanten Daten vollständig, unverfälscht und unmittelbar zur Verfügung stehen. Für die digitale Archivierung spricht ferner, daß zahlreiche Daten bereits jetzt, zumindest aber künftig, von vornherein in digitaler Form vorliegen, wie Röntgenbilder, Ultraschallaufnahmen, EKG, EEG, Arzberichte usw.

Weitere vorteilhafte und bevorzugte Ausgestaltungen der Erfindung ergeben sich durch die Ansprüche und deren Kombinationen.

Nachfolgend werden Einzelheiten der Erfindung unter Bezugnahme auf die Zeichnungen näher angegeben, in denen:
Figur 1 eine schematische Darstellung von Systemkomponenten eines Daten-Archivierungssystems zeigt,
Figuren 2a - 2c tabellarische Darstellungen möglicher Aufteilungen der Speichereinrichtungen sind, und
Figur 3 ein Blockschaltbild des Aufbaus von Figur 1 ist.

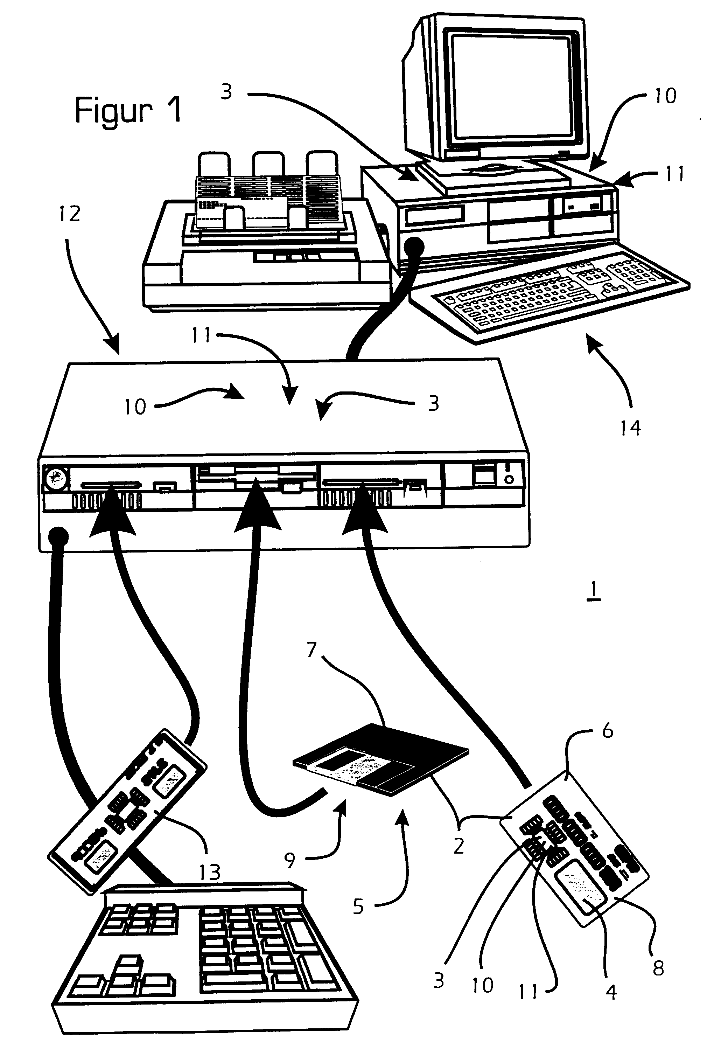

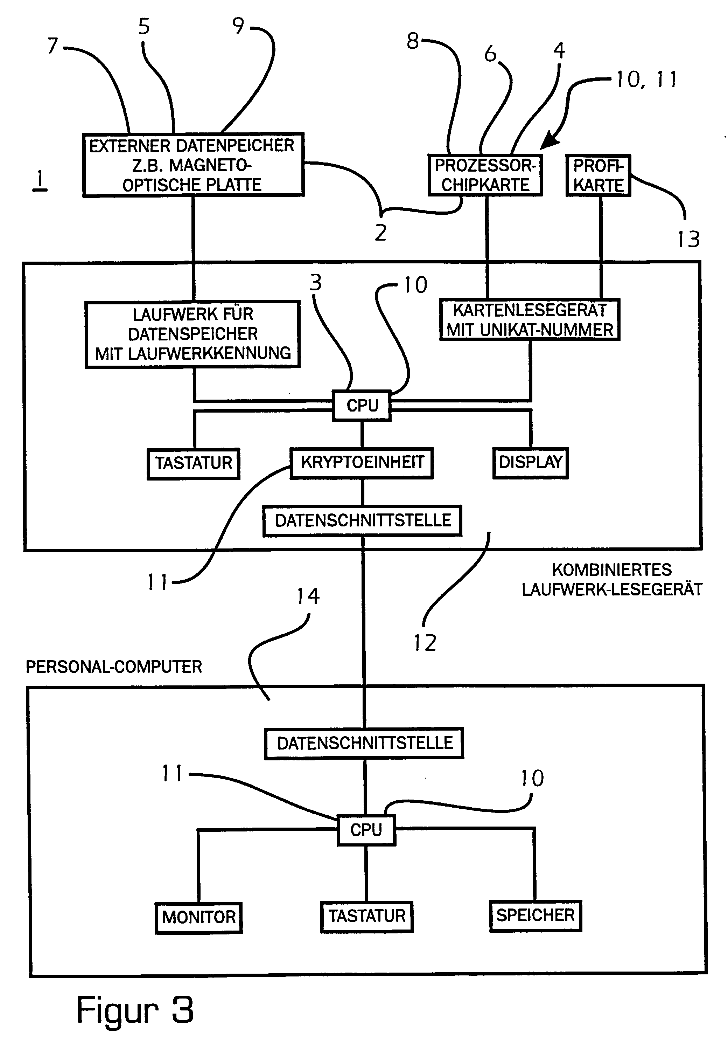

In den Figuren 1 und 3 ist ein Daten-Archivierungssystem 1 gezeigt. Das System 1 enthält transportable oder mobile, persönliche Speichereinrichtungen 2 zum Speichern und Aufbewahren von Daten beim Inhaber. Die Speichereinrichtungen enthalten erste und zweite getrennte Datenträger 8 und 9, die einzelne Speichervorrichtungen als ersten und zweiten Teil 6 bzw. 7 der Speichereinrichtungen 2 bilden.

Zum Zugreifen auf die einzelnen Datenträger 8, 9 ist ein Zugriffsgerät 12 gezeigt, das ein kombiniertes Lesegerät und Laufwerk für die beiden Datenträger 8, 9 darstellt. Ferner ist das Zugriffsgerät 12 auch ausgelegt, eine Berechtigungskarte 13 zu lesen.

Der erste Datenträger 6 ist eine Prozessor-Chipkarte und dient sowohl zur Speicherung kleinerer Datenmengen, beispielsweise eines Noffallausweises, von Übersichtsdaten und/oder Behandlungsdaten des Inhabers und von Nachweisdaten erfolgter Speicherzugriffe, als auch als Bestandteil von Berechtigungsprüfeinrichtungen 3, wofür die Prozessoreinrichtungen 10 der Karte eingesetzt werden. Wie insbesondere der Figur 3 deutlich zu entnehmen ist, sind im Zugriffsgerät 12 weitere Prozessoreinrichtungen 10 vorgesehen, die ebenfalls Bestandteil der Berechtigungsprüfeinrichtungen 3 zur Feststellung einer Zugriffsberechtigung sind.

Der zweite Datenträger 7 ist eine magneto-optische Platte und ermöglicht so eine Speicherung großer Datenmengen zur Speicherung von Detaildaten. Außerdem ist durch die Prozessoreinrichtungen 10 eine Steuerung vorgesehen, um zu vermeiden, daß die Prozessor-Chipkarte speichermäßig voll ist und keine weiteren Daten mehr aufnehmen kann, wofür Daten von der Prozessor-Chipkarte immer wieder auf die magneto-optische Platte ausgelagert werden.

Die den Speichereinrichtungen 2 zugeordneten Berechtigungsprüfeinrichtungen 3 dienen der Datensicherheit, da sie dafür sorgen, daß nur in Abhängigkeit von einer positiven Berechtigungs- und/oder Authentizitätserkennung ein Zugriff auf zumindest einige der auf den persönlichen Speichereinrichtungen 2 gespeicherten Daten freigegeben werden kann. Bei dem gezeigten Ausführungsbeispiel sind hierfür die einzelnen Prozessoreinrichtungen 10 und später noch genauer angegebene Kennungen zuständig.

Die Berechtigungsprüfeinrichtungen 3 des gezeigten Ausführungsbeispiels sind so ausgeführt, daß sie einen turnusmäßigen Kennungswechsel einer Anwender- und/oder Gerätekennung zur Berechtigungs- und/oder Authentizitätserkennung berücksichtigen und eine Authentizitäts- und/oder Berechtigungsprüfung von Anwender, Zugriffsgeräten und Speichereinrichtungen 2 für einen beabsichtigten Zugriff durchführen. Weiterhin können Schreibzugriffe auf die Speichereinrichtungen 2 dort nur nach Eingabe einer Signatur zusammen mit dieser abgespeichert werden, wobei diese Signatur durch eine Anwender- bzw. Zugriffsgerätekennung automatisch unter Nutzung des RSA-Verfahrens erzeugt wird.

Das gezeigte Archivierungssystem 1 ist so ausgelegt, daß nur in Verbindung mit dem ersten Teil 6 der Speichereinrichtungen 2 auf den zweiten Teil 7 der Speichereinrichtungen 2 zugegriffen werden kann. Es sind auch mehrere verschiedene Prüf- bzw. Berechtigungsstufen vorgesehen, um den Zugriff auf verschiedene Datenbereiche zu regeln.

In einer ersten Prüfstufe wird nur eine Lesegerätekennung überprüft, um einen Zugriff auf Notfalldaten zuzulassen. In dieser oder einer höheren Prüfstufe kann eventuell eine Berechtigungskarte für den Zugriff auf Daten erforderlich sein, die eine Übersicht über weitere persönliche Daten des Inhabers der Chipkarte geben. Eine weitere Prüfstufe kann dadurch realisiert werden, daß auf die auf dem zweiten Teil 7 der persönlichen Speichereinrichtungen 2 gespeicherten Daten, wie Detaildaten, nur in Abhängigkeit von einer positiven Berechtigungs- und/oder Authentizitätserkennung mittels der Berechtigungsprüfeinrichtungen 3 zugreifbar ist. Eine solche Kennung kann z.B. folgende Informationen alleine oder kombiniert berücksichtigen: eine Lesegerätekennung, eine Laufwerkskennung, eine Datenträgerkennung, eine Anwenderkennung, eine Inhaberkennung. Insbesondere die beiden letzteren können auch durch PINs (persönliche Identifizierungsnummern) oder Geheimzahlen o.ä. realisiert werden, wobei verschiedene Eingaben zu unterschiedlichen Berechtigungsstufen führen können. Die einzelnen Prüfstufen der Berechtigungsprüfeinrichtungen 3 können beispielsweise auch dafür genutzt werden, um für Schreib- und Lesezugriffe jeweils unterschiedliche Berechtigungen zu vergeben.

Im Zusammenhang mit dem Kartenlesegerät wird durch die Berechtigungsprüfeinrichtungen 3 die Authentizität der Prozessor-Chipkarte überprüft. Dabei können z.B. gefälschte Karten abgewiesen werden. Ebenso wird anhand der Prozessor-Chipkarte mittels der Berechtigungsprüfeinrichtungen 3 eine Überprüfung des Kartenlesegeräts durchgeführt, so daß nicht autorisierte Lesegeräte überhaupt keinen Zugriff auf die Daten der Prozessor-Chipkarte und auch des Massenspeichers erhalten. Eine ähnliche gegenseitige Autorisierungsüberprüfung kann auch für die magneto-optische Platte und das entsprechende Laufwerk vorgesehen sein.

Im einzelnen werden die Identifikationsmerkmale des Kartenlesegeräts (z.B. Unikat-Nr.) und des Laufwerks für die magneto-optische Platte (z.B. Laufwerkkennung) erfaßt und die entsprechenden Daten auf oder in dem Speicher der Prozessor-Chipkarte gespeichert, sobald das Lesegerät oder das Laufwerk auf gespeicherte Daten zugreift. Sollte die Kapazität des entsprechenden Speichers auf der Prozessor-Chipkarte nicht ausreichen, werden ältere Daten auf die MO-Platte ausgelagert oder gelöscht. Durch eine Erfassung der Benutzeridentifikation (Anwender- und Inhaberkennungen) wird getrennt für die Prozessor-Chipkarte und die MO-Platte jeglicher Zugriff auf die gespeicherten Daten erfaßt. Alternativ oder auch ergänzend zu anderen Identifikationsmerkmalen, wie der Lesegerätekennung oder einer Benutzerkennung, kann eine Berechtigungskarte zur Autorisierungsprüfung benötigte Daten bereitstellen, die den Benutzer ausweisen und ebenfalls gespeichert werden können, um dort, wo verschiedene Benutzer auf das gleiche Kartenlesegerät zugreifen, wie z.B. in einem Rettungswagen, eine einfache, schnelle und sichere Berechtigungserkennung zu ermöglichen.

Die umfangreichen Daten auf dem zweiten Teil 7 der Speichereinrichtungen 2, also beispielsweise Bilddaten auf der MO-Platte, können nur dann gelesen werden, wenn ein Benutzer beispielsweise über entsprechende Programmteile verfügt, was aber erst nach positiver Authentizitätsprüfung möglich ist.

Als weiteren Sicherheitsaspekt enthält das Zugriffsgerät 12 in dem gezeigten Beispiel Kryptoeinrichtungen 11, die für eine Entschlüsselung und Verschlüsselung bei Lese- bzw. Schreibzugriffen auf die Teile 6 und 7 der Speichereinrichtungen 2 sorgen und beispielsweise zur Bearbeitung von großen Datenmengen zumindest einen als Coprozessor ausgelegten Kryptoprozessor enthalten. Die Kryptoeinrichtungen 11 sind den Speichereinrichtungen 2 vorgeschaltet und werden betriebsmäßig mittels der Berechtigungsprüfeinrichtungen 3 zur Bearbeitung von Zugriffen auf die Speichereinrichtungen 2 freigegeben. Die zwischen einzelnen Speichervorrichtungen bzw. deren Zugriffseinrichtungen 12 und den Rechnereinrichtungen 14 transferierten Daten werden durch die Kryptoeinheit ent- bzw. verschlüsselt, wobei letztere ebenfalls erst nach positiv ausgefallener Authentizitätsprüfung in Funktion tritt. Beim Aufruf einer Datei auf der MO-Platte wird diese automatisch entschlüsselt. Umgekehrt erfolgt eine automatische Verschlüsselung einer Datei, wenn sie auf die MO-Platte geschrieben wird. Die entsprechenden Dateinamen sind beim System 1 ohne die Freigabe durch die Berechtigungsprüfeinrichtungen 3 nicht lesbar und auf der Prozessor-Chipkarte hinterlegt. Die hohe Datentransferrate kann durch die eigenständige Kryptoeinheit bewältigt werden.

Der erste Teil der Speichereinrichtungen 2 bzw. die erste Speichervorrichtung 6 ist nicht auf die vorbeschriebenen Ausführung beschränkt, sondern kann eine magnetische, magneto-optische oder optische Speicherfläche oder ein Speicherchip insbesondere auf oder in einer vorzugsweise etwa scheckkartengroßen Kunststoffkarte als Datenträger 8 sein. Ebenfalls ist die Ausgestaltung des zweiten Teils der Speichereinrichtungen 2 bzw. der zweiten Speichervorrichtung 7 nicht auf eine magneto-optische Platte beschränkt, sondern kann jeglicher elektronische, magnetische, magneto-optische oder optische Massenspeicher insbesondere in Form einer 2,5 oder 3,5 Zoll großen Diskette oder einer PCMCIA-Einheit als Datenträger 9 sein.

Die Speichereinrichtungen 2 sind jeweils durch ein zum Hinzufügen und/oder Ändern von Daten wiederbeschreibbares Speichermedium 4, 5 entsprechend den Datenträgern 8, 9 gebildet. Für bestimmte Anwendungszwecke sind wenigstens Teile der beiden beschreibbaren Speichermedien 4, 5 schreibgeschützt oder schreibschützbar. Hierdurch können beispielsweise unveränderliche Eintragungen vor versehentlichen Überschreibungen geschützt werden. Auch Bereiche des Speichermediums 5 des zweiten Datenträgers können aus dem gleichen Grund schreibgeschützt oder schreibschützbar sein, um z.B. nachträgliche Änderungen in der Zugriffsdokumentation auszuschließen.

Die Prozessoreinrichtungen 10 erfüllen neben ihrer Aufgabe zur Berechtigungsüberprüfung noch die Steuerung von zumindest Teilen der Speichereinrichtungen 2 und/oder die wenigstens teilweise Steuerung von Zugriffen auf letztere.

Nicht gesondert gezeigt sind Kopierschutzeinrichtungen, Druckausgabeschutzeinrichtungen und Ausgabebeschränkungseinrichtungen, die ein unerwünschtes Kopieren, Drucken oder andersartiges Ausgeben von den Speichereinrichtungen 2 entnommenen Daten verhindern, wenn diese entsprechenden Einrichtungen nicht durch einen geeignete Berechtigungsnachweis den Berechtigungsprüfeinrichtungen gegenüber neutralisiert, d.h. abgeschaltet sind.

Statt des oder zusätzlich zum gezeigten Zugriffsgerät 12 können für die einzelnen Datenträger 8 und 9 sowie die Berechtigungskarte 13 gesonderte Zugriffsgeräte vorgesehen sein, beispielsweise persönliche, mobile und/oder stationäre Zugriffsgeräte. Das Zugriffsgerät 12 ist sowohl für die Prozessor-Chipkarte als auch die magneto-optische Platte in Minidisk-Form für einen Schreib- und/oder Lesezugriff ausgelegt.

Jedes einsetzbare Zugriffsgerät 12 enthält zweckmäßigerweise entsprechend den Formaten von die Speichereinrichtungen 2 aufnehmenden Datenträgern Kartenlesegeräte für z.B. Magnetstreifen- oder Chipkarten, Diskettenlaufwerke für z.B. magnetische, magneto-optische oder optische Disketten, CD-ROM-Laufwerke und/oder Aufnahmen für PCMCIA-Einheiten. Auch bei getrennter Ausführung der Zugriffsgeräte 12 für die ersten und zweiten Teile 6, 7 der Speichereinrichtungen 2 können diese Geräte 12 zusammengeschaltet und über diese Zusammenschaltung von den Berechtigungsprüfeinrichtungen 3 ein entsprechender Berechtigungsnachweis erfaßt werden.

Zur Erstellung von Sicherungskopien, die bei Verlust der Speichereinrichtungen 2 einen vollständigen Datenverlust verhindern, lassen die Zugriffsgeräte 12 direkt oder die Berechtigungsprüfeinrichtungen 3 ohne Berechtigungsnachweis eine Erstellung von Sicherungskopien der transportablen, persönlichen Speichereinrichtungen 2 zu. Hierfür können z.B. flüchtige Speichereinrichtungen in dem entsprechenden Zugriffsgerät 12 enthalten sein, um Daten zum Kopieren zwischenzuspeichern.

Wie den Figuren 1 und 3 weiter zu entnehmen ist, ist zum Ansteuern des Zugriffsgeräts 12 eine Rechnereinrichtung 14 in Form eines PCs vorgesehen. Diese Rechnereinrichtungen 14 dienen ferner als Anzeige- und Ein-/Ausgabegeräte von Daten sowohl für die Berechtigungsprüfeinrichtungen 3 als auch die Speichereinrichtungen 2, um Daten zu suchen, auszugeben und einzugeben.

Das in der Figur 1 dargestellte Zugriffsgerät 12 ist über eine Leitung mit einer nicht näher bezeichneten eigenen Tastatur und über eine weitere Leitung an geeigneten Datenschnittstellen mit den Rechnereinrichtungen 14 verbunden. Die Rechnertastatur und/oder die Zugriffsgerätetastatur kann zum Eingeben von PINs u.ä. verwendet werden können. Ferner ist das Zugriffsgerät 12 mit nicht näher bezeichneten Displayeinrichtungen versehen.

Der erste Datenträger 8 ist gleichzeitig eine Krankenversicherungskarte und das gesamte System 1 dier zur Archivierung von Patientendaten einschließlich schriftlicher und graphischer Unterlagen beim Inhaber. Jedoch können auch andere Daten mit diesem oder ähnlichen Systemen archiviert werden, wie beispielsweise Finanzdaten, Versicherungsdaten, Reisedaten u.v.m.

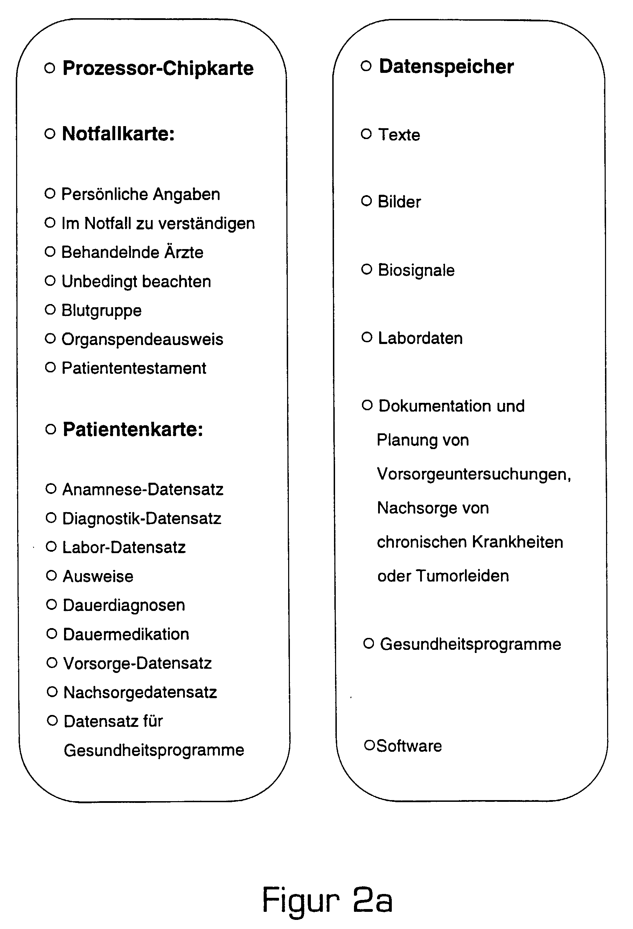

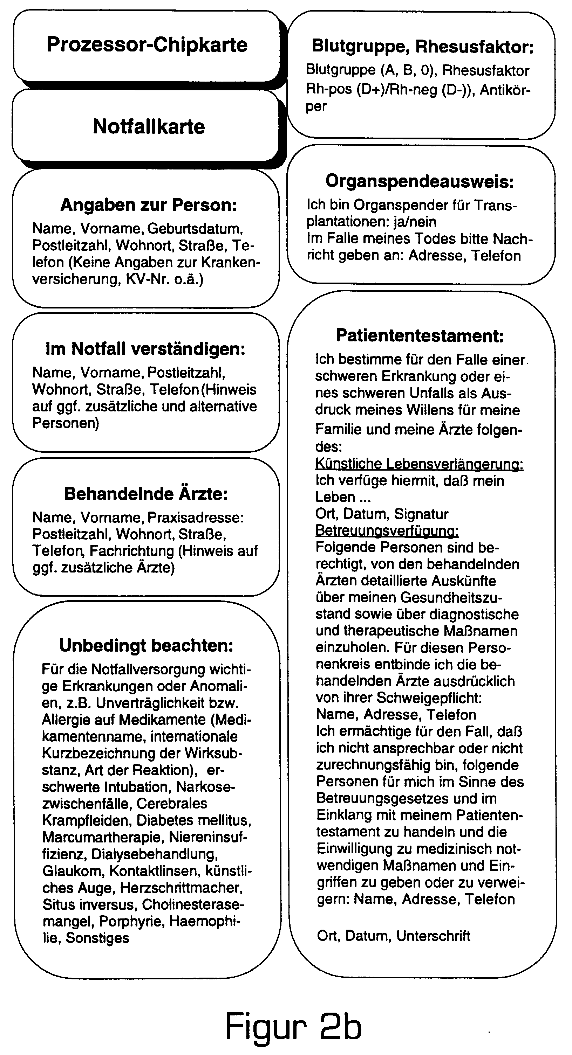

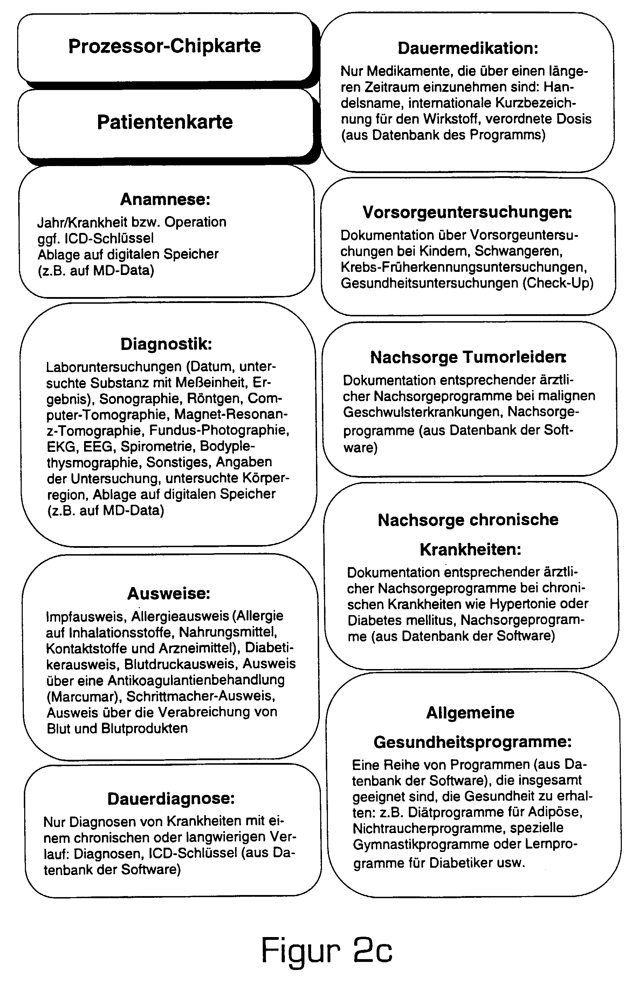

Für den Anwendungsfall des Patientendaten-Archivierungssystems ist in den Figuren 2a - 2c tabellarisch eine Speicherorganisation dargestellt. Die Figur 2a zeigt die Organisationsstruktur der beiden Datenträger, die Figur 2b gibt Aufschluß über den Inhalt der enthaltenen Notfallkarte und die Figur 2c erläutert den Inhalt der Patientenkarte, wobei die verschiedenen Speicherbereiche direkt bezeichnet sind, so daß keine weiteren Erläuterungen zu ihrem Verständnis erforderlich sind. In der Praxis können einzelne Felder und Einträge über die Rechnereinrichtungen 14 mittels Menü- oder Fenstertechnik aufgerufen werden.

Wenn vorstehend auf ein Patientendaten-Archivierungssystem, Patientendaten und allgemein den Medizinbereich Bezug genommen wurde, so ist dies nur exemplarisch zu verstehen. Die Erfindung betrifft ein Daten-Archivierungssystem, das durch die in den Ansprüchen angegebenen Merkmale und Merkmalskombinationen bestimmt und nicht auf einzelne Ausführungsbeispiele der Beschreibung beschränkt ist. So können z.B. mehr als zwei (separate) Speichereinrichtungsteile vorgesehen sein, wobei beispielsweise eine einzige Prozessorkarte mit mehreren MO-Platten für je ein eigenes Sachgebiet zusammenarbeiten kann. Insbesondere sind alle durch die Merkmalsformulierungen in den Ansprüchen enthaltenen Ausführungsmöglichkeiten der Erfindung ohne Einschränkungen und mit den dem Fachmann geläufigen Modifikationen und Substitutionen im Umfang der Erfindung abgedeckt.

### Bezugszeichenliste

- 1: Daten-Archivierungssystem
- 2: Speichereinrichtungen
- 3: Berechtigungsprüfeinrichtungen
- 4, 5: Speichermedium
- 6: erster Teil der Speichereinrichtungen
- 7: zweiter Teil der Speichereinrichtungen
- 8, 9: erste und zweite Datenträger
- 10: Prozessoreinrichtungen
- 11: Kryptoeinrichtungen
- 12: Zugriffsgeräte
- 13: Berechtigungskarte, Arzt- oder Rettungsdienstkarte
- 14: Rechnereinrichtungen

## Patentansprüche

1. Daten-Archivierungssystem
- mit transportablen, eine erste Speichervorrichtung (6) als einen ersten Teil und eine separate zweite Speichervorrichtung (7) als einen zweiten Teil enthaltenden Speichereinrichtungen (2) und
- mit den Speichereinrichtungen (2) zugeordneten Berechtigungsprüfeinrichtungen (3), mittels denen nur in Abhängigkeit von einer positiven Berechtigungs- und/oder Authentizitätserkennung ein Zugriff auf zumindest einige der auf den Speichereinrichtungen (2) gespeicherten Daten freigebbar ist,
**dadurch gekennzeichnet,**
- daß die erste und die zweite Speichervorrichtung (6,7) und darauf gespeicherte Daten einem einzigen Inhaber zugeordnet sind zum Aufbewahren der Daten beim Inhaber und
- daß auf die auf der zweiten Speichervorrichtung (7) gespeicherten Daten nur in Verbindung mit der dem Inhaber zugeordneten ersten Speichervorrichtung (6) und nur in Abhängigkeit von einer positiven Berechtigungs- und/oder Authentizitätserkennung mittels der Berechtigungsprüfeinrichtungen (3) zugreifbar ist.

2. Daten-Archivierungssystem nach Anspruch 1,
**dadurch gekennzeichnet,**
daß mittels den Berechtigungsprüfeinrichtungen (3) eine Mehrzahl von Prüfstufen ausführbar ist.

3. Daten-Archivierungssystem nach Anspruch 1 oder 2,
**dadurch gekennzeichnet,**
daß zumindest ein Teil der Speichereinrichtungen (2) durch ein zum Hinzufügen und/oder Ändern von Daten beschreibbares und insbesondere wiederbeschreibbares Speichermedium (4, 5) gebildet ist.

4. Daten-Archivierungssystem nach Anspruch 3,
**dadurch gekennzeichnet,**
daß die Berechtigungsprüfeinrichtungen (3) zum Freigeben eines Lese- und/oder Schreibzugriffs auf das beschreibbare Speichermedium (4, 5) in Abhängigkeit von einer insbesondere entsprechenden Berechtigungs- und/oder Authentizitätserkennung ggf. in einer zugehörigen Prüfstufe ausgelegt sind.

5. Daten-Archivierungssystem nach Anspruch 3 oder 4,
**dadurch gekennzeichnet,**
daß zumindest ein Teil des beschreibbaren Speichermediums (4, 5) schreibgeschützt oder schreibschützbar ist.

6. Daten-Archivierungssystem nach einem der vorhergehenden Ansprüche,
**dadurch gekennzeichnet,**
daß der erste Teil (6) und der zweite Teil (7) der Speichereinrichtungen (2) unterschiedliche Speicherkapazitäten enthalten.

7. Daten-Archivierungssystem nach einem der Ansprüche 1-6,
**dadurch gekennzeichnet,**
daß der erste, insbesondere kapazitätsmäßig kleinste Teil (6) der Speichereinrichtungen (2) ein Ausweis-, Identifikations- oder Stammdatenspeicherteil ist.

8. Daten-Archivierungssystem nach einem der Ansprüche 1 bis 7,
**dadurch gekennzeichnet,**
daß auf die auf dem ersten Teil (6) der persönlichen Speichereinrichtungen (2) gespeicherten Daten, wie Ausweis-, Identifikations- oder Stammdaten, unabhängig von den Berechtigungsprüfeinrichtungen (3) oder mit einer positiven Berechtigungs- und/oder Authentizitätserkennung in einer ersten Prüfstufe der Berechtigungsprüfeinrichtungen (3) zugreifbar ist.

9. Daten-Archivierungssystem nach einem der Ansprüche 1 bis 8,
**dadurch gekennzeichnet,**
daß der zweite, relativ zum ersten Teil (6) kapazitätsmäßig größere Teil (7) der Speichereinrichtungen (2) ein Detaildatenspeicherteil ist.

10. Daten-Archivierungssystem nach einem der Ansprüche 1 bis 9,
**dadurch gekennzeichnet,**
daß die separaten Speichervorrichtungen (6, 7) auf verschiedenen ersten bzw. zweiten Datenträgern (8, 9) angeordnet sind.

11. Daten-Archivierungssystem nach einem der Ansprüche 1 bis 10,
**dadurch gekennzeichnet,**
daß der erste Teil der Speichereinrichtungen (2) bzw. die erste Speichervorrichtung (6) eine magnetische, magneto-optische oder optische Speicherfläche oder ein Speicherchip insbesondere auf oder in einer vorzugsweise etwa scheckkartengroßen Kunststoffkarte als Datenträger (8) ist.

12. Daten-Archivierungssystem nach einem der Ansprüche 1 bis 11,
**dadurch gekennzeichnet,**
daß der zweite Teil der Speichereinrichtungen (2) bzw. die zweite Speichervorrichtung (7) ein elektronischer, magnetischer, magneto-optischer oder optischer Massenspeicher insbesondere in Form einer 2,5 oder 3,5 Zoll großen Diskette oder einer PCMCIA-Einheit als Datenträger (9) ist.

13. Daten-Archivierungssystem nach einem der vorhergehenden Ansprüche,
**dadurch gekennzeichnet,**
daß zumindest einem Teil der Speichereinrichtungen (2) zugeordnete und/oder vorzugsweise wenigstens einen Teil der Berechtigungsprüfeinrichtungen (3) bildende Prozessoreinrichtungen (10) vorgesehen sind, die vorzugsweise auf dem Datenträger (8), wie z.B. einer Kunststoffkarte, angeordnet sind und insbesondere einen Prozessor enthalten.

14. Daten-Archivierungssystem nach den Ansprüchen 11 und 13,
**dadurch gekennzeichnet,**
daß die Prozessoreinrichtungen (10) zur Steuerung von zumindest Teilen der Speichereinrichtungen (2) und/oder zur wenigstens teilweisen Steuerung von Zugriffen auf letztere und/oder zumindest als Teil der Berechtigungsprüfeinrichtungen (3) ausgelegt sind.

15. Daten-Archivierungssystem nach einem der vorhergehenden Ansprüche,
**dadurch gekennzeichnet,**
daß den Speichereinrichtungen (2) Kryptoeinrichtungen (11) vorgeschaltet sind, deren Betrieb insbesondere mittels der Berechtigungsprüfeinrichtungen (3) zur Bearbeitung von Zugriffen auf die Speichereinrichtungen (2) freigebbar ist.

16. Daten-Archivierungssystem nach Anspruch 13 oder 14 und Anspruch 15,
**dadurch gekennzeichnet,**
daß die Kryptoeinrichtungen (11) in den Prozessoreinrichtungen (10) enthalten sind und vorzugsweise einen Kryptoprozessor aufweisen.

17. Daten-Archivierungssystem nach einem der vorhergehenden Ansprüche,
**dadurch gekennzeichnet,**
daß die Berechtigungsprüfeinrichtungen (3) ausgelegt sind, Gerätekennungen von Zugriffsgeräten (12) für die Handhabung der Speichereinrichtungen (2) in eine Prüfung zur Berechtigungs- und/oder Authentizitätserkennung, insbesondere zur Erfüllung einer Prüfstufe, einzubeziehen.

18. Daten-Archivierungssystem nach einem der vorhergehenden Ansprüche,
**dadurch gekennzeichnet,**
daß die persönlichen Speichereinrichtungen (2) Kennungen aufweisen, die von den Berechtigungsprüfeinrichtungen (3) in eine Prüfung zur Berechtigungsund/oder Authentizitätserkennung, insbesondere zur Erfüllung einer Prüfstufe, einbeziehbar sind.

19. Daten-Archivierungssystem nach einem der vorhergehenden Ansprüche,
**dadurch gekennzeichnet,**
daß die Berechtigungsprüfeinrichtungen (3) ausgelegt sind, Benutzer- und/oder Inhaberidentifikationen von zumindest einem Anwender bzw. dem Inhaber, dessen Daten in den Speichereinrichtungen (2) archiviert sind, in eine Prüfung zur Berechtigungs- und/oder Authentizitätserkennung, insbesondere zur Erfüllung einer Prüfstufe und ggf. weiterer Prüfstufen, zur Freigabe eines Zugriffs auf zumindest einige der auf den persönlichen Speichereinrichtungen (2) gespeicherten Daten einzubeziehen.

20. Daten-Archivierungssystem nach Anspruch 19,
**dadurch gekennzeichnet,**
daß die Berechtigungsprüfeinrichtungen (3) ausgelegt sind, eine Anwenderkennung, wie eine Benutzer- bzw. Inhaberidentifikation über manuelle und/oder elektronische Eingaben, wie beispielsweise von einer Berechtigungskarte (13), durchzuführen.

21. Daten-Archivierungssystem nach einem der vorhergehenden Ansprüche,
**dadurch gekennzeichnet,**
daß durch die Berechtigungsprüfeinrichtungen (3) zugelassene Zugriffe auf den Speichereinrichtungen (2) mit den dafür relevanten Daten, wie Gerätekennungen von Zugriffsgeräten (12) und/oder Benutzer- und/oder Inhaberidentifikationen, insbesondere auf den Speichereinrichtungen (2) selbst dokumentierbar sind.

22. Daten-Archivierungssystem nach einem der vorhergehenden Ansprüche,
**dadurch gekennzeichnet,**
daß persönliche Zugriffsgeräte (12) vorgesehen sind, mittels denen vom Inhaber, dessen Daten in den Speichereinrichtungen (2) archiviert sind, und/oder von Anwendern der Speichereinrichtungen (2) in Abhängigkeit von einer insbesondere entsprechenden Berechtigungs- und/oder Authentizitätserkennung ggf. in einer zugehörigen Prüfstufe auf zumindest einige der archivierten Daten zum Zweck der Information, Datenänderung/-ergänzung und/oder Weitergabe auch über Datenfernübertragung zugreifbar ist.

23. Daten-Archivierungssystem nach einem der vorhergehenden Ansprüche,
**dadurch gekennzeichnet,**
daß mobile und/oder stationäre Zugriffsgeräte (12) vorgesehen sind, mittels denen von Anwendern in Abhängigkeit von einer insbesondere entsprechenden Berechtigungs- und/oder Authentizitätserkennung ggf. in einer zugehörigen Prüfstufe auf zumindest einige der archivierten Daten zum Zweck der Information und/oder Datenänderung/-ergänzung zugreifbar ist.

24. Daten-Archivierungssystem nach Anspruch 22 oder 23,
**dadurch gekennzeichnet,**
daß die Zugriffsgeräte (12) für einen Schreib- und/oder Lesezugriff zumindest auf einige der auf den persönlichen Speichereinrichtungen (2) gespeicherten Daten in Abhängigkeit von einer insbesondere entsprechenden Berechtigungs- und/oder Authentizitätserkennung ggf. in einer zugehörigen Prüfstufe ausgelegt sind.

25. Daten-Archivierungssystem nach einem der Ansprüche 22 bis 24,
**dadurch gekennzeichnet,**
daß die Zugriffsgeräte (12) Gerätekennungen aufweisen, die von den Berechtigungsprüfeinrichtungen (3) zur Prüfung einer Berechtigungs- und/oder Authentizitätserkennung, insbesondere zur Erfüllung einer Prüfstufe, erfaßbar sind, und daß die Gerätekennung vorzugsweise durch eine in zumindest ein Zugriffsgerät (12) eingebbare Anwenderkennung aktivierbar ist.

26. Daten-Archivierungssystem nach einem der Ansprüche 22 bis 25,
**dadurch gekennzeichnet,**
daß zum Ansteuern der Zugriffsgeräte (12) herkömmliche Rechnereinrichtungen (14) vorgesehen sind.

27. Daten-Archivierungssystem nach einem der Ansprüche 22 bis 26,
**dadurch gekennzeichnet,**
daß die Zugriffsgeräte (12) entsprechend den Formaten von die Speichereinrichtungen (2) aufnehmenden Datenträgern Kartenlesegeräte für z.B. Magnetstreifen- oder Chipkarten, Diskettenlaufwerke für z.B. magnetische, magneto-optische oder optische Disketten, CD-ROM-Laufwerke und/oder Aufnahmen für PCMCIA-Einheiten enthalten.

28. Daten-Archivierungssystem nach einem der Ansprüche 22 bis 27,
**dadurch gekennzeichnet,**
daß zumindest zwei Zugriffsgeräte (12) zur Zusammenschaltung von entsprechenden Datenträgern (8, 9) der Speichereinrichtungen (2) kombiniert sind und diese Zusammenschaltung von den Berechtigungsprüfeinrichtungen (3) zum Berechtigungsnachweis oder ggf. als eine erste Prüfstufe erfaßbar ist.

29. Daten-Archivierungssystem nach einem der Ansprüche 22 bis 28,
**dadurch gekennzeichnet,**
daß die Zugriffsgeräte (12) ausgelegt sind, ohne Berechtigungsnachweis oder ggf. in einer ersten Prüfstufe Sicherungskopien der transportablen, persönlichen Speichereinrichtungen (2) zu erstellen.

30. Daten-Archivierungssystem nach einem der vorhergehenden Ansprüche,
**dadurch gekennzeichnet,**
daß es zum Speichern und Aufbewahren von Patientendaten, einschließlich schriftlicher und graphischer Unterlagen, beim Inhaber ausgelegt ist.

31. Daten-Archivierungssystem nach Anspruch 30,
**dadurch gekennzeichnet,**
daß der erste Teil (6) der Speichereinrichtungen (2) einen Notfalldatenspeicherbereich enthält, in dem Verwaltungsdaten und die Daten eines Notfallausweises, eines Organspendeausweises, Notfalltestaments u.ä. des Inhabers speicherbar sind und auf den ein Zugriff zumindest soweit durch die Berechtigungsprüfeinrichtungen (3) frei oder mit nur einer Arzt- oder Rettungsdienstkennung, die vorzugsweise über eine spezielle Arzt- oder Rettungsdienstkarte (13) oder eine Arzt-/Rettungsdienst-Zugriflsgerätekennung in die Berechtigungsprüfeinrichtungen (3) eingebbar ist, oder mit nur einer Inhaberkennung freigebbar ist.

32. Daten-Archivierungssystem nach Anspruch 30 oder 31,
**dadurch gekennzeichnet,**
daß der erste Teil (6) der Speichereinrichtungen (2) einen Übersichtsdatenspeicherbereich enthält, in dem eine Auflistung wichtiger anamnestischer Daten, einschließlich einer ggf. erfolgten Verabreichung von Blut oder Blutprodukten und eine Dokumentation wichtiger Gesundheits-/Krankheitsdaten, einschließlich Allergie-, Impf-, Röntgen-, Schrittmacher-, Diabetiker-, Medikamentendaten u.ä., analog einer ärztlichen Patientenkarte speicherbar sind und auf den ein Zugriff zumindest soweit durch die Berechtigungsprüfeinrichtungen (3) frei oder einzeln oder in Kombination mit einer Arzt- oder Rettungsdienstkennung, die vorzugsweise über eine spezielle Arzt- oder Rettungsdienstkarte (13) oder eine Arzt-/Rettungsdienst-Zugriffsgerätekennung in die Berechtigungsprüfeinrichtungen (3) eingebbar ist, mit einer Inhaberkennung und/oder einer Krankenkassenkennung freigebbar ist.

33. Daten-Archivierungssystem nach den Ansprüchen 30 oder 31 oder 32,
**dadurch gekennzeichnet,**
daß der erste Teil (6) der Speichereinrichtungen (2) einen Behandlungsdatenspeicherbereich enthält, in dem ein vorgegebener zeitlicher Rahmen und eine vorgegebene inhaltliche Struktur von Vorsorgeuntersuchungen sowie Untersuchungen im Rahmen der Nachsorge chronischer und/oder bösartiger Erkrankungen speicherbar sind und auf den ein Zugriff zumindest soweit durch die Berechtigungsprüfeinrichtungen (3) einzeln oder in Kombination mit einer Arztkennung, die vorzugsweise über eine spezielle Arztkarte (13) oder eine Arzt-Zugriffsgerätekennung in die Berechtigungsprüfeinrichtungen (3) eingebbar ist, mit einer Inhaberkennung und/oder einer Krankenkassenkennung freigebbar ist.

34. Daten-Archivierungssystem nach einem der Ansprüche 30 bis 33,
**dadurch gekennzeichnet,**
daß der erste Teil (6) der Speichereinrichtungen (2) einen Nachweisdatenspeicherbereich enthält, in dem zumindest eine vorgebbare Anzahl und/oder Art von Zugriffen auf die Speichereinrichtungen (2) insbesondere einschließlich der jeweils erfolgten vorzugsweise individuellen Berechtigungs- und/oder Authentizitätserkennung und vorgenommenen Zugriffsart im einzelnen speicherbar ist und auf den ein Zugriff zumindest soweit durch die Berechtigungsprüfeinrichtungen (3) einzeln oder in Kombination mit einer Arztkennung, die vorzugsweise über eine spezielle Arztkarte (13) oder eine Arzt-Zugriffsgerätekennung in die Berechtigungsprüfeinrichtungen (3) eingebbar ist, mit einer Inhaberkennung und/oder einer Krankenkassenkennung freigebbar ist.

35. Daten-Archivierungssystem nach einem der Ansprüche 30 bis 34,
**dadurch gekennzeichnet,**
daß der zweite Teil (7) der Speichereinrichtungen (2) einen Dokumentationsbereich enthält, in dem alle Originalunterlagen von Arztberichten, Zeugnissen, Röntgen-, Ultraschall-, Computertomographieabbildungen usw., Biosignale, wie EKG- und EEG-Daten etc., direkt oder als Graphiken u.ä. speicherbar sind und auf den ein Zugriff zumindest soweit durch die Berechtigungsprüfeinrichtungen (3) einzeln oder in Kombination mit einer Arztkennung, die vorzugsweise über eine spezielle Arztkarte (13) oder eine Arzt-Zugriffsgerätekennung in die Berechtigungsprüfeinrichtungen (3) eingebbar ist, mit einer Inhaberkennung und/oder einer Krankenkassenkennung freigebbar ist.

36. Daten-Archivierungssystem nach einem der Ansprüche 30 bis 35,
**dadurch gekennzeichnet,**
daß der zweite Teil (7) der Speichereinrichtungen (2) einen Nachweisdatenspeicherbereich enthält, in dem zumindest eine vorgebbare Anzahl und/oder Art von Zugriffen oder alle Zugriffe auf die Speichereinrichtungen (2) insbesondere einschließlich der jeweils erfolgten vorzugsweise individuellen Berechtigungs- und/oder Authentizitätserkennung und vorgenommenen Zugriffsart im einzelnen speicherbar ist und auf den ein Zugriff zumindest soweit durch die Berechtigungsprüfeinrichtungen (3) einzeln oder in Kombination mit einer Arztkennung, die vorzugsweise über eine spezielle Arztkarte (13) oder eine Arzt-Zugriffsgerätekennung in die Berechtigungsprüfeinrichtungen (3) eingebbar ist, mit einer Inhaberkennung und/oder einer Krankenkassenkennung freigebbar ist.

37. Daten-Archivierungssystem nach den Ansprüchen 34 und 36,
**dadurch gekennzeichnet,**
daß der Nachweisdatenspeicherbereich im ersten Teil (6) der Speichereinrichtungen (2) schieberegisterartig aufgebaut ist, so daß sein ältester Inhalt zur insbesondere endgültigen Abspeicherung in den Nachweisdatenspeicherbereich des zweiten Teils (7) der Speichereinrichtungen (2) zur Platzschaffung für einen Neueintrag im Nachweisdatenspeicherbereich im ersten Teil (6) der Speichereinrichtungen (2) verschiebbar ist.

38. Daten-Archivierungssystem nach einem der vorhergehenden Ansprüche,
**dadurch gekennzeichnet,**
daß die Speichereinrichtungen (2) ganz oder teilweise Bestandteil einer Krankenversicherungskarte, eines Personalausweises oder einer Kontokarte sind.

39. Daten-Archivierungssystem nach einem der vorhergehenden Ansprüche,
**dadurch gekennzeichnet,**
daß mittels den Berechtigungsprüfeinrichtungen (3) ein insbesondere turnusmäßiger Kennungswechsel einer Anwender- und/oder Gerätekennung zur Berechtigungs- und/oder Authentizitätserkennung berücksichtigbar ist.

40. Daten-Archivierungssystem nach einem der vorhergehenden Ansprüche,
**dadurch gekennzeichnet,**
daß mittels den Berechtigungsprüfeinrichtungen (3) eine Authentizitäts- und/oder Berechtigungsprüfung von Anwender, Zugriffsgeräten (12) und/oder Speichereinrichtungen (2) für einen beabsichtigten Zugriff durchführbar ist.

41. Daten-Archivierungssystem nach einem der vorhergehenden Ansprüche,
**dadurch gekennzeichnet,**
daß die Speichereinrichtungen (2) eine Mehrzahl von Bereichen enthalten, denen unterschiedliche Zugriffssicherungen, wie Passwörter, PINs, Berechtigungskarten (13) o.ä. zuordenbar sind.

42. Daten-Archivierungssystem nach einem der vorhergehenden Ansprüche,
**dadurch gekennzeichnet,**
daß Schreibzugriffe auf die Speichereinrichtungen (2) dort nur nach Eingabe einer Signatur zusammen mit dieser abspeicherbar sind, und daß letztere vorzugsweise durch eine Anwender- und/oder Zugriffsgerätekennung insbesondere automatisch erzeugbar ist.

## Claims

1. Data archiving system
- having transportable storage devices (2) containing a first storage device (6) as a first part and a separate second storage device (7) as a second part and
- having authorization checking devices (3) that are associated with the storage devices (2) and by means of which access to at least some of the data stored on the storage devices (2) can be enabled only as a function of a positive authorization and/or authentication,
characterized
- in that the first and the second storage device (6, 7) and data stored thereon are assigned to a single owner for the preservation of the data at the owner and
- in that the data stored on the second storage device (7) are accessible only in conjunction with the first storage device (6) assigned to the owner and only as a function of a positive authorization and/or authentication by means of the authorization checking devices (3) .

2. Data archiving system according to Claim 1, characterized in that a plurality of checking stages may be carried out by means of the authorization checking devices (3).

3. Data archiving system according to Claim 1 or 2, characterized in that at least part of the storage devices (2) is formed by a storage medium (4, 5) that can be written and in particular rewritten in order to append and/or change data.

4. Data archiving system according to Claim 3, characterized in that the authorization checking devices (3) are designed to enable a read and/or write access to the writable storage medium (4, 5) as a function of an in particular appropriate authorization and/or authentication, optionally in an associated checking stage.

5. Data archiving system according to Claim 3 or 4, characterized in that at least part of the writable storage medium (4, 5) is write-protected or can be write-protected.

6. Data archiving system according to one of the preceding claims, characterized in that the first part (6) and the second part (7) of the storage devices (2) contain different storage capacities.

7. Data archiving system according to one of Claims 1-6, characterized in that the first part (6), in particular the smallest part in terms of capacity, of the storage devices (2) is an identity, identification or master data storage part.

8. Data archiving system according to one of Claims 1 to 7, characterized in that the data stored on the first part (6) of the personal storage devices (2), such as identity, identification or master data, are accessible independently of the authorization checking devices (3) or with a positive authorization and/or authentication in a first checking stage of the authorization checking devices (3).

9. Data archiving system according to one of Claims 1 to 8, characterized in that the second part (7), greater in terms of capacity in relation to the first part (6), of the storage devices (2) is a detail data storage part.

10. Data archiving system according to one of Claims 1 to 9, characterized in that the separate storage devices (6, 7) are arranged on different first and second data carriers (8, 9).

11. Data archiving system according to one of Claims 1 to 10, characterized in that the first part of the storage devices (2) or the first storage device (6) is a magnetic, magneto-optical or optical storage area or a storage chip, in particular on or in a preferably cheque-card-sized plastic card as data carrier (8).

12. Data archiving system according to one of Claims 1 to 11, characterized in that the second part of the storage devices (2) or the second storage device (7) is an electronic, magnetic, magneto-optical or optical mass store, in particular in the form of a 2.5 or 3.5 inch floppy disk or a PCMCIA unit as data carrier (9).

13. Data archiving system according to one of the preceding claims, characterized in that processor devices (10) assigned to at least one part of the storage devices (2) and/or preferably forming at least part of the authorization checking devices (3) are provided, which are preferably arranged on the data carrier (8), such as for example a plastic card, and in particular contain a processor.

14. Data archiving system according to Claims 11 and 13, characterized in that the processor devices (10) are designed for the control of at least parts of the storage devices (2) and/or for the at least partial control of accesses to the latter and/or at least as part of the authorization checking devices (3).

15. Data archiving system according to one of the preceding claims, characterized in that connected upstream of the storage devices (2) are crypto devices (11), whose operation can be enabled in particular by means of the authorization checking devices (3) in order to process accesses to the storage devices (2).

16. Data archiving system according to Claim 13 or 14 and Claim 15, characterized in that the crypto devices (11) are contained in the processor devices (10) and preferably have a crypto processor.

17. Data archiving system according to one of the preceding claims, characterized in that the authorization checking devices (3) are designed to incorporate device codes of access devices (12) for handling the storage devices (2) in a check for the purpose of authorization and/or authentication, in particular to fulfil a checking stage.

18. Data archiving system according to one of the preceding claims, characterized in that the personal storage devices (2) have codes which can be incorporated by the authorization checking devices (3) into a check for the purpose of authorization and/or authentication, in particular to fulfil a checking stage.

19. Data archiving system according to one of the preceding claims, characterized in that the authorization checking devices (3) are designed to incorporate user and/or owner identifications of at least one user or the owner, whose data are archived in the storage devices (2), into a check for the purpose of authorization and/or authentication, in particular to fulfil a checking stage and, optionally, further checking stages, in order to enable access to at least some of the data stored on the personal storage devices (2).

20. Data archiving system according to Claim 19, characterized in that the authorization checking devices (3) are designed to carry out a user identification, such as a user or owner identification, via manual and/or electronic inputs, such as for example from an authorization card (13).

21. Data archiving system according to one of the preceding claims, characterized in that accesses permitted by the authorization checking devices (3) to the storage devices (2) having the data relevant for this, such as device codes from access devices (12) and/or user and/or owner identifications, can themselves be documented, in particular on the storage devices (2).

22. Data archiving system according to one of the preceding claims, characterized in that personal access devices (12) are provided, by means of which the owner, whose data are archived in the storage devices (2), and/or users of the storage devices (2), as a function of an in particular appropriate authorization and/or authentication, optionally in an associated checking stage, can access at least some of the archived data for the purposes of information, data modification/supplementation and/or onward transmission, including via remote data transmission.

23. Data archiving system according to one of the preceding claims, characterized in that mobile and/or stationary access devices (12) are provided, by means of which, as a function of an in particular appropriate authorization and/or authentification, optionally in an associated checking stage, users can access at least some of the archived data for the purpose of information and/or data modification/supplementation.

24. Data archiving system according to Claim 22 or 23, characterized in that the access devices (12) are designed for write and/or read access, at least to some of the data stored on the personal storage devices (2), as a function of an in particular appropriate authorization and/or authentication, optionally in an associated checking stage.

25. Data archiving system according to one of Claims 22 to 24, characterized in that the access devices (12) have device codes, which can be registered by the authorization checking devices (3) in order to check an authorization and/or authentication, in particular to fulfil a checking stage, and in that the device code can be activated preferably by a user code that can be entered into at least one access device (12).

26. Data archiving system according to one of Claims 22 to 25, characterized in that conventional computer devices (14) are provided in order to drive the access devices (12).

27. Data archiving system according to one of Claims 22 to 26, characterized in that the access devices (12), corresponding to the formats of the data carriers receiving the storage devices (2), contain card-reading devices for, for example, magnetic strip or chip cards, disk drives for, for example, magnetic, magneto-optical or optical floppy disks, CD-ROM drives and/or receptacles for PCMCIA units.

28. Data archiving system according to one of Claims 22 to 27, characterized in that at least two access devices (12) are combined in order to interconnect corresponding data carriers (8, 9) of the storage devices (2), and this interconnection can be registered by the authorization checking devices (3) to prove authorization or, optionally, as a first checking stage.

29. Data archiving system according to one of Claims 22 to 28, characterized in that the access devices (12) are designed to create back-up copies of the transportable, personal storage devices (2) without proof of authorization or, optionally, in a first checking stage.

30. Data archiving system according to one of the preceding claims, characterized in that it is designed for the storage and preservation of patient data, including written and graphical documents, with the owner.

31. Data archiving system according to Claim 30, characterized in that the first part (6) of the storage devices (2) contains an emergency data storage area, in which administration data and the data of an emergency card, an organ-donor card, emergency will and the like of the owner can be stored, and to which access can be enabled, at least to some extent by the authorization checking devices (3), freely or only using a doctor or emergency services code, which can preferably be input via a special doctor or emergency services card (13) or a doctor/emergency services access device code into the authorization checking device (3), or only using an owner code.

32. Data archiving system according to Claim 30 or 31, characterized in that the first part (6) of the storage devices (2) contains an overview data storage area, in which a listing of important case-history data, including any administration of blood or blood products carried out and documentation of important health/illness data, including allergy, inoculation, X-ray, pacemaker, diabetic, medication data and the like, can be stored in a manner analogous to a patient's medical card, and to which access can be enabled, at least to some extent by the authorization checking devices (3), freely or individually or in combination with a doctor or emergency services code, which can preferably be entered via a special doctor or emergency services card (13) or a doctor/emergency services access device code into the authorization checking devices (3), with an owner code and/or a health insurance code.

33. Data archiving system according to Claims 30 or 31 or 32, characterized in that the first part (6) of the storage devices (2) contains a treatment data storage area, in which a predetermined time frame and a predetermined content structure of medical check-ups and examinations in the context of follow-up care of chronic and/or malignant diseases can be stored, and to which access is enabled, at least to some extent by the authorization checking devices (3), individually or in combination with a doctor code, which can preferably be entered via a special doctor card (13) or a doctor access device code into the authorization checking devices (3), with an owner code and/or a health insurance code.

34. Data archiving system according to one of Claims 30 to 33, characterized in that the first part (6) of the storage devices (2) contains a verification data storage area, in which at least a predefineable number and/or type of accesses to the storage devices (2), in particular including the preferably individual authorization and/or authentication carried out in each case and the type of access performed can be stored in detail, and to which access can be enabled, at least to some extent by the authorization checking devices (3), individually or in combination with a doctor code, which can preferably be entered via a special doctor card (13) or a doctor access device code into the authorization checking devices (3), with an owner code and/or a health insurance code.

35. Data archiving system according to one of Claims 30 to 34, characterized in that the second part (7) of the storage devices (2) contains a documentation area, in which all the original documents of medical records, reports, X-rays, ultrasound and computer tomography images and so on, biosignals, such as ECG and EEG data, etc., can be stored directly or in the form of graphics and the like, and to which access can be enabled, at least to some extent by the authorization checking devices (3), individually or in combination with a doctor code, which can preferably be entered via a special doctor card (13) or a doctor access device code into the authorization checking devices (3), with an owner code and/or a health insurance code.

36. Data archiving system according to one of Claims 30 to 35, characterized in that the second part (7) of the storage devices (2) contains a verification data storage area, in which at least a predefineable number and/or type of accesses or all accesses to the storage devices (2), in particular including the preferably individual authorization and/or authentication carried out in each case and the type of access performed can be stored in detail, and to which access can be enabled, at least to some extent by the authorization checking devices (3), individually or in combination with a doctor code, which can preferably be entered via a special doctor card (13) or a doctor access device code into the authorization checking devices (3), with an owner code and/or a health insurance code.

37. Data archiving system according to Claims 34 and 36, characterized in that the verification data storage area in the first part (6) of the storage devices (2) is constructed like a shift register, so that its oldest contents can be shifted, in particular for final storage in the verification data storage area of the second part (7) of the storage devices (2), in order to create space for a new entry in the verification data storage area in the first part (6) of the storage devices (2).

38. Data archiving system according to one of the preceding claims, characterized in that the storage devices (2) are wholly or partly a component of a health insurance card, a personal identification or an ATM card.

39. Data archiving system according to one of the preceding claims, characterized in that a code change, in particular one made cyclically, of a user and/or device code can be taken into account by means of the authorization checking devices (3) for the purpose of authorization and/or authentication.

40. Data archiving system according to one of the preceding claims, characterized in that by means of the authorization checking devices (3), an authenticity and/or authorization check of the user, access devices (12) and/or storage devices (2) can be carried out for an intended access.

41. Data archiving system according to one of the preceding claims, characterized in that the storage devices (2) contain a plurality of areas, to which different access safeguards, such as passwords, PINs, authorization cards (13) or the like can be allocated.

42. Data archiving system according to one of the preceding claims, characterized in that write accesses to the storage devices (2) can be stored there together with a signature only after the entry of this signature, and that the latter can be generated, in particular automatically, preferably by means of a user and/or access device code.

## Revendications

1. Système d'archivage de données équipé
- de systèmes de mémoire (2) contenant un premier dispositif de mémoire (6) en tant que première partie et un second dispositif de mémoire distinct (7) en tant que seconde partie et
- de dispositifs de contrôle de vérification (3) associés aux dispositifs de mémoire (2), à l'aide desquels uniquement, en fonction d'une reconnaissance positive de correction et/ou d'authenticité, un accès est permis à au moins certaines des données mémorisées dans les dispositifs de mémoire (2), caractérisé en ce que
- le premier et le second dispositif de mémoire (6, 7) et des données mémorisées dans ceux-ci sont associés à un seul propriétaire en vue de la conservation des données concernant le propriétaire, et
- les données mémorisées sur le second dispositif de mémoire (7) sont accessibles uniquement en liaison avec le premier dispositif de mémoire (6) associé au propriétaire et en fonction d'une reconnaissance positive de correction et/ou d'authenticité au moyen des dispositifs de contrôle de correction (3).

2. Système d'archivage de données selon la revendication 1, caractérisé en ce qu'une pluralité d'étapes de contrôle peut être réalisée au moyen des dispositifs de contrôle de correction (3).

3. Système d'archivage de données selon la revendication 1 ou 2, caractérisé en ce qu'au moins une partie des dispositifs de mémoire (2) est formée par un support de mémoire (4, 5) pouvant être écrit et en particulier réécrit en vue de l'adjonction et/ou de la modification de données.

4. Système d'archivage de données selon la revendication 3, caractérisé en ce que les dispositifs de contrôle de correction (3) en vue de l'autorisation d'un accès de lecture et/ou d'écriture sur le support de mémoire (4, 5) pouvant être écrit en fonction d'une reconnaissance en particulier correspondante de correction et/ou d'authenticité sont disposés éventuellement dans un étage de contrôle associé.

5. Système d'archivage de données selon la revendication 3 ou 4, caractérisé en ce qu'au moins une partie du support de mémoire devant être écrit (4, 5) est ou peut être protégé vis-à-vis d'une inscription.

6. Système d'archivage de données selon l'une quelconque des revendications précédentes, caractérisé en ce que la première partie (6) et la seconde partie (7) comportent des dispositifs de mémoire (2) de capacités de mémoire différentes.

7. Système d'archivage de données selon l'une quelconque des revendications 1 à 6, caractérisé en ce que la première partie (6) en particulier de capacité la plus faible des dispositifs de mémoire (2) est une partie de mémoire de données d'identité, d'identification ou d'origine.

8. Système d'archivage de données selon l'une quelconque des revendications 1 à 7, caractérisé en ce que des données mémorisées sur la première partie (6) des dispositifs de mémoire personnels (2), telles que des données d'identité, d'identification ou d'origine, sont accessibles indépendamment des dispositifs de contrôle de correction (3) ou avec une reconnaissance positive de correction et/ou d'authenticité dans un premier étage de contrôle des dispositifs de contrôle de correction (3).

9. Système d'archivage de données selon l'une quelconque des revendications 1 à 8, caractérisé en ce que la seconde partie (7) de capacité plus grande comparativement à la première partie (6) des dispositifs de mémoire (1) est une partie de mémoire de données de détail.

10. Système d'archivage de données selon l'une quelconque des revendications 1 à 9, caractérisé en ce que les dispositifs de mémoire distincts (6, 7) sont disposés sur différents premier ou second supports de données (8, 9).

11. Système d'archivage de données selon l'une quelconque des revendications 1 à 10, caractérisé en ce que la première partie des dispositifs de mémoire (2) ou le premier dispositif de mémoire (6) est une surface de mémoire magnétique, magnéto-optique ou optique ou une puce de mémoire en particulier sur ou dans une carte de matière plastique, de préférence environ de la grandeur d'une carte de crédit en tant que support de données (8).

12. Système d'archivage de données selon l'une quelconque des revendications 1 à 11, caractérisé en ce que la seconde partie des dispositifs de mémoire (2) ou le second dispositif de mémoire (7) est une mémoire de masse électronique, magnétique, magnéto-optique ou optique sous la forme d'une disquette de 2,5 ou 3,5 pouces ou d'une unité PCMCIA en tant que support de données (9).

13. Système d'archivage de données selon l'une quelconque des revendications précédentes, caractérisé en ce que des processeurs (10), associés au moins à une partie des dispositifs de mémoire (2) et/ou formant de préférence au moins une partie des dispositifs de contrôle de correction (3), sont prévus, qui sont disposés de préférence sur le support de données (8), comme par exemple une carte en matière plastique et contiennent en particulier un processeur.

14. Système d'archivage de données selon les revendications 11 et 13, caractérisé en ce que les processeurs (10), en vue de la commande d'au moins deux parties des dispositifs de mémoire (2) et/ou en vue de la commande au moins partielle d'accès, sont disposés sur ces derniers et/ou au moins en tant que partie des dispositifs de contrôle de correction (3).

15. Système d'archivage de données selon l'une quelconque des revendications précédentes, caractérisé en ce qu'en amont des dispositifs de mémoire (2) sont disposés des crypteurs (11), dont le fonctionnement peut être autorisé en particulier au moyen des dispositifs de contrôle de correction (3) en vue du traitement d'accès aux dispositifs de mémoire (2).

16. Système d'archivage de données selon les revendications 13 ou 14 et la revendication 15, caractérisé en ce que les crypteurs (11) sont contenus dans les processeurs (10) en comportent de préférence un processeur de cryptage.

17. Système d'archivage de données selon l'une quelconque des revendications précédentes, caractérisé en ce que les dispositifs de contrôle de correction (3) sont prévus afin de comporter des caractérisations d'appareils d'accès (12) en vue du fonctionnement des dispositifs de mémoire (12) dans un contrôle en vue de la reconnaissance de correction et/ou d'authenticité, en particulier en vue de la réalisation d'une étape de contrôle.

18. Système d'archivage de données selon l'une quelconque des revendications précédentes, caractérisé en ce que les dispositifs de mémoire personnels (2) comportent des caractérisations, qui peuvent être incorporées par les dispositifs de contrôle de correction (3) dans un contrôle en vue de la reconnaissance de correction et/ou d'authenticité, en particulier en vue de la réalisation d'une étape de contrôle.

19. Système d'archivage de données selon l'une quelconque des revendications précédentes, caractérisé en ce que les dispositifs de contrôle de correction (3) sont prévus pour des identifications d'utilisateur et/ou de propriétaire d'au moins un utilisateur ou du propriétaire dont les données sont archivées dans des dispositifs de mémoire (2), en vue d'une incorporation dans un contrôle en vue de la reconnaissance de la correction et/ou de l'authenticité, en particulier en vue de la réalisation d'une étape de contrôle et éventuellement d'autres étapes de contrôle, en vue du dégagement d'un accès à au moins certaines données mémorisées dans les dispositifs de mémoire personnels (2).

20. Système d'archivage de données selon la revendication 19, caractérisé en ce que les dispositifs de contrôle de correction (3) sont prévus afin de réaliser une caractérisation d'application, comme une identification d'utilisateur ou de propriétaire par l'intermédiaire d'entrées manuelles et/ou électroniques, comme par exemple par une carte de correction (13).

21. Système d'archivage de données selon l'une quelconque des revendications précédentes, caractérisé en ce que des accès autorisés par des dispositifs de contrôle de correction (3) aux dispositifs de mémoire (2) avec les données pertinentes à cet effet, comme des caractérisations d'appareils d'accès (12) et/ou des identifications d'utilisateur et/ou de propriétaire, peuvent être eux-mêmes documentés en particulier sur les dispositifs de mémoire (2).

22. Système d'archivage de données selon l'une quelconque des revendications précédentes, caractérisé en ce que des appareils d'accès personnels (12) sont prévus, au moyens desquels est accessible par le propriétaire, dont les données sont archivées dans les dispositifs de mémoire (2), et/ou par des utilisateurs des dispositifs de mémoire (2) en fonction d'une reconnaissance correspondante en particulier de correction et/ou d'authenticité, éventuellement au cours d'une étape de contrôle associée à au moins certaines des données archivées, dans un but d'information, de modification/complément de données et/ou restitution également par télétransmission de données.

23. Système d'archivage de données selon l'une quelconque des revendications précédentes, caractérisé en ce que des appareils d'accès mobiles et/ou stationnaires (12) sont prévus, au moyen desquels est accessible par des utilisateurs en fonction d'une reconnaissance correspondante particulière de correction et/ou d'authenticité éventuellement au cours d'une étape de contrôle associée au moins certaines des données archivées dans un but d'information et/ou de modification/complément de données.

24. Système d'archivage de données selon la revendication 22 ou 23, caractérisé en ce que les appareils d'accès (12) pour un accès d'écriture et/ou de lecture au moins à certaines des données mémorisées dans les dispositifs de mémoire personnels (2) sont prévus en fonction d'une reconnaissance correspondante particulière de correction et/ou d'authenticité éventuellement au cours d'une étape de contrôle associée.

25. Système d'archivage de données selon l'une des revendications 22 à 24, caractérisé en ce que les appareils d'accès (12) présentent des caractéristiques qui peuvent être détectées par les dispositifs de contrôle de correction (3) en vue du contrôle d'une reconnaissance de correction et/ou d'authenticité, en particulier en vue de l'accomplissement d'une étape de contrôle, et en ce que la caractéristique de l'appareil peut être activée de préférence par une caractéristique d'utilisateur pouvant être entrée dans au moins un appareil d'accès (12).

26. Système d'archivage de données selon l'une des revendications 22 à 25, caractérisé en ce qu'en vue de la commande des appareils d'accès (12) sont prévus des calculateurs classiques (14).

27. Système d'archivage de données selon l'une des revendications 22 à 26, caractérisé en ce que les appareils d'accès (12) correspondant aux formats de support de données recevant les dispositifs de mémoire (2) contiennent des appareils de lecture de carte pour, par exemple, des cartes à bande magnétique ou à puce, des mécanismes pour, par exemple, des disquettes magnétiques, magnéto-optiques ou optiques, des mécanismes de CD-ROM et/ou des logements pour des unités PCMCIA.

28. Système d'archivage de données selon l'une quelconque des revendications 22 à 27, caractérisé en ce qu'au moins deux appareils d'accès (12) sont combinés en vue de la commutation de supports de données correspondants (8, 9) des dispositifs de mémoire (2) et cette commutation peut être détectée par les dispositifs de contrôle de correction (3) en tant que preuve de correction ou éventuellement en tant que première étape de contrôle.

29. Système d'archivage de données selon l'une quelconque des revendications 22 à 28, caractérisé en ce que les appareils d'accès (12) sont prévus afin d'établir, sans preuve de correction ou éventuellement au cours d'une première étape de contrôle, des copies de sécurité des dispositifs de mémoire personnels transportables (2).

30. Système d'archivage de données selon l'une quelconque des revendications précédentes, caractérisé en ce qu'il est réalisé en vue de la mémorisation et de la conservation de données de malades, y compris des documents écrits et graphiques, concernant le propriétaire.

31. Système d'archivage de données selon la revendication 30, caractérisé en ce que la première partie (6) des dispositifs de mémoire (2) contient un emplacement de mémoire de données d'urgence, dans lequel des données administratives et les données d'une preuve en cas d'urgence, d'une preuve de don d'organe, de testament d'urgence et autres du propriétaire peuvent être mémorisées et auquel un accès peut être obtenu au moins par l'intermédiaire des dispositifs de contrôle de correction (3) librement ou uniquement par l'intermédiaire d'un service médical ou de secours, qui peut être introduit de préférence par l'intermédiaire d'une carte spéciale d'un service médical ou de secours (13) ou une connaissance d'appareil d'accès du service médical ou de secours dans les dispositifs de contrôle de correction (3), ou uniquement par l'intermédiaire de la connaissance du propriétaire.

32. Système d'archivage de données selon la revendication 30 ou 31, caractérisé en ce que la première partie (6) des dispositifs de mémoire (2) contient un emplacement de mémoire de données d'ensemble, dans lequel peut être mémorisée une liste de données ineffaçables, importantes, y compris une transfusion éventuelle de sang ou de produits sanguins et une documentation de données de santé/maladie importantes, y compris des données concernant l'allergie, la vaccination, la radiographie, un stimulateur cardiaque, le diabète, des médicaments, et autres, analogue à une carte de malade et auquel un accès peut être obtenu au moins par l'intermédiaire des dispositifs de contrôle de correction (3) librement ou séparément ou en combinaison avec une connaissance du service médical ou de secours, qui peut être introduite par l'intermédiaire d'une carte spéciale médicale ou de secours (13) ou par l'intermédiaire d'une caractéristique des appareils d'accès de service médical ou de secours dans les dispositifs de contrôle de correction (3), avec une identification du propriétaire et/ou d'une caisse maladie.

33. Système d'archivage de données selon les revendications 30 ou 31 ou 32, caractérisé en ce que la première partie (6) des dispositifs de mémoire (2) contient un emplacement de mémoire de données de traitement, dans lequel un cadre temporel prédéterminé et une structure de contenu prédéterminée de recherches de soins antérieurs ainsi que de recherches dans le cadre de soins postérieurs de maladies chroniques et/ou graves peuvent être mémorisées, et auquel un accès peut être obtenu au moins par l'intermédiaire des dispositifs de contrôle de correction (3) isolément ou en combinaison avec une connaissance médicale, qui peut être introduite de préférence par l'intermédiaire d'une carte médicale particulière (13) ou une caractérisation d'appareils d'accès médicaux, dans les dispositifs de contrôle de correction (3), peut être obtenu à l'aide d'une connaissance du propriétaire et/ou d'une connaissance de la caisse maladie.

34. Système d'archivage de données selon l'une quelconque des revendications 30 à 33, caractérisé en ce que la première partie (6) des dispositifs de mémoire (2) contient un emplacement de mémoire de données de preuve, dans lequel au moins un nombre prédéterminé et/ou un type d'accès aux dispositifs de mémoire (2), en particulier y compris la caractérisation de préférence individuelle de correction et/ou d'authenticité et de type d'accès effectué, peut être mémorisé isolément et auquel un accès peut être obtenu au moins par l'intermédiaire des dispositifs de contrôle de correction (3) isolément ou en combinaison avec une connaissance médicale, qui peut être entrée de préférence par l'intermédiaire d'une carte médicale particulière (13) ou par l'intermédiaire d'une caractérisation d'appareils d'accès médical dans les dispositifs de contrôle de correction (3), peut être obtenu à l'aide de la connaissance du propriétaire et/ou de la connaissance d'une caisse maladie.

35. Système d'archivage de données selon l'une quelconque des revendications 30 à 34, caractérisé en ce que la seconde partie (7) des dispositifs de mémoire (2) contient un emplacement de documentation dans lequel peuvent être mémorisés tous les documents originaux de rapports médicaux, témoins, images radiograhiques, échographiques, tomographiques par calculateur, etc., biosignaux, comme des données d'électrocardiogramme et d'électroencéphalogramme, etc., directement ou en tant que graphiques et autres et auquel un accès peut être obtenu au moins par l'intermédiaire des dispositifs de contrôle de correction (3) isolément ou en combinaison avec une caractérisation médicale, qui peut être obtenu de préférence par l'intermédiaire d'une carte médicale particulière (13) ou d'une connaissance d'appareils d'accès médical d'accès dans les dispositifs de contrôle de correction (3), à l'aide de la connaissance du propriétaire et/ou de la connaissance d'une caisse maladie.

36. Système d'archivage de données selon l'une quelconque des revendications 30 à 35, caractérisé en ce que la seconde partie (7) des dispositifs de mémoire (2) contient un emplacement de mémoire de données de preuve, dans lequel peut être mémorisé un nombre prédéterminé et/ou un type d'accès ou tous les accès aux dispositifs de mémoire (2) en particulier y compris la reconnaissance effectuée de préférence individuellement de correction et/ou d'authenticité et le type d'accès effectué isolément et auquel un accès peut être obtenu au moins par l'intermédiaire des dispositifs de contrôle de correction (3) isolément ou en combinaison avec une caractérisation médicale, qui peut être introduite de préférence par l'intermédiaire d'une carte médicale particulière (13) ou d'une caractérisation d'appareils d'accès médical dans les dispositifs de contrôle de correction (3), peut être obtenu à l'aide d'une connaissance du propriétaire et/ou d'une connaissance de la caisse maladie.

37. Système d'archivage de données selon les revendications 34 et 36, caractérisé en ce que l'emplacement de mémoire de données de preuve dans la première partie (6) des dispositifs de mémoire (2) est structuré à la manière d'un registre à décalage, de sorte que son plus ancien contenu peut être déplacé en vue de la mémorisation en particulier définitive dans l'emplacement de mémoire de données de preuve de la deuxième partie (7) des dispositifs de mémoire (2) en vue de créer de la place pour une nouvelle entrée dans l'emplacement de mémoire de données de preuve dans la première partie (6) du dispositif de mémoire (2).

38. Système d'archivage de données selon l'une quelconque des revendications précédentes, caractérisé en ce que les dispositifs de mémoire (2) sont en totalité ou partiellement un constituant d'une carte d'assurance maladie, d'une carte d'identité personnelle ou d'une carte de compte.

39. Système d'archivage de données selon l'une quelconque des revendications précédentes, caractérisé en ce qu'au moyen des dispositifs de contrôle de correction (3), un changement de caractérisation en particulier par roulement d'une caractérisation d'utilisateur et/ou d'appareil peut être pis en compte en vue d'une reconnaissance de correction et/ou d'authenticité.

40. Système d'archivage de données selon l'une quelconque des revendications précédentes, caractérisé en ce qu'au moyen des dispositifs de contrôle de correction (3), un contrôle d'authenticité et/ou de correction d'utilisateur, d'appareils d'accès (12) et/ou de dispositifs de mémoire (2) peut être effectué en vue d'un accès envisagé.

41. Système d'archivage de données selon l'une quelconque des revendications précédentes, caractérisé en ce que les dispositifs de mémoire (2) contiennent une pluralité d'emplacements, auxquels peuvent être associées différentes sécurités d'accès, telles que des mots de passe, des numéros individuels personnels, des cartes de correction (13) ou autres.

42. Système d'archivage de données selon l'une quelconque des revendications précédentes, caractérisé en ce que des accès d'écriture aux dispositifs de mémoire (2) ne peuvent y être mémorisés qu'après introduction d'une signature conjointement avec celle-ci, et que cette dernière peut être générée en particulier automatiquement de préférence par une caractérisation d'utilisateur et/ou d'appareils d'accès.
